# EUROPEAN PATENT APPLICATION

(11) **EP 3 369 459 A1**
(43) Date of publication of application: **05.09.2018**
(21) Application number: 17020083.6
(22) Date of filing: 01.03.2017
(51) Int. Cl.: A61Q 17/04

(54) **KIT OF COSMETIC SKINCARE COMPOSITIONS**

(71) Applicant: The Boots Company PLC, Nottingham NG90 1BS (GB)
(72) Inventor: James, Leanne Marie, LOUGHBOROUGH, Leicestershire LE11 1LX (GB); O'Connor, Clare Helena, ILKESTON, Derbyshire DE7 6HG (GB); Rowney, Jamie, NOTTINGHAM, NG5 2BW (GB); Elms, Christopher John, NOTTINGHAM, NG13 0FH (GB)
(74) Representative: Goodier, Claire-Louise

(57) **Abstract**

According to the present invention there is provided a kit of cosmetic compositions, the kit comprising:
1) a cleansing composition, the cleansing composition comprising: (i) a cosmetically acceptable carrier; (ii) one or more surfactants; (iii) one or more anti-inflammatory agent; (iv) one or more antioxidant agent selected from polyphenolic antioxidant agents and antioxidant vitamins; (v) one or more skin-soothing agent;
2) a skin balancing composition, the skin balancing composition comprising: (i) a cosmetically acceptable carrier; (ii) *Ophiopogon japonicas* extract; (iii) one or more antioxidant agent selected from polyphenolic antioxidant agents and antioxidant vitamins; (iv) one or more sebum control agent selected from *Rosa canina* extract and sphingosine compounds;
3) a daytime protective composition, the daytime protective composition comprising: (i) a cosmetically acceptable carrier; (ii) one or more UV-filter; (iii) one or more antioxidant agent selected from polyphenolic antioxidant agents and antioxidant vitamins; (iv) one or more skin-soothing agent; and
4) an overnight treatment composition, the overnight treatment composition comprising: (i) a cosmetically acceptable carrier; (ii) one or more polyphenolic antioxidant agent; (iii) one or more antioxidant vitamin; (iv) one or more skin-soothing agent.

## Description

### Field of the invention

The present invention relates to a kit of cosmetic compositions providing improved skin protection and methods of cosmetic treatment using said kit.

### Background of the invention

The skin is the first line of defence, serving as a barrier between us and the environment. The skin is a complex organ consisting of three layers: the epidermis, dermis and hypodermis.

The epidermis is the outermost layer, which itself is made up of several layers. The outermost portion of the epidermis, known as the stratum corneum, is relatively waterproof and, when undamaged, prevents most bacteria, viruses, and other foreign substances from entering the body. It also prevents the loss of moisture, heat and other important constituents of the body.

Most of the cells (90-95%) in the epidermis are keratinocytes. They originate from proliferating keratinocyte stem cells in the deepest layer of the epidermis called the basal layer. Resulting keratinocytes further divide and differentiate and slowly migrate up toward the surface of the epidermis as mature cells. Once the keratinocytes reach the stratum corneum at the skin surface they are dead and no longer multiplying and are gradually shed and replaced by newer cells pushed up from below.

The skin is subject to constant attack by a variety of both exogenous and endogenous insults. Exogenous insults include those arising from the environment such as ultraviolet radiation (UVA and UVB), infra-red and visible light, atmospheric pollution (including cigarette smoke) and/or harsh chemicals including surfactants in cosmetic formulations. Such environmental factors may either directly or indirectly result in skin damage by the generation of reactive species and free radicals, for example superoxide anions, hydrogen peroxide, hydroxyl ions, peroxyl ions, ozone, singlet oxygen, sulphur oxide, nitrogen oxide, carbon monoxide, alkoxyl ion, peroxynitrite and heavy metals. Reactive oxygen species (ROS), reactive carbonyl species (RCS) and reactive nitrogen species (RNS) need to be particularly considered. Endogenous insults can also result in skin damage, for example hormonal fluctuations (e.g. cortisol and adrenaline hormones), aging and other biochemical changes from within the skin.

With respect to atmospheric pollution (including cigarette smoke), polycyclic aromatic hydrocarbons (PAHs) are key pollutants that cause skin damage through a number of different mechanisms including increased melanocyte activation, increased sebum oxidation and mitochondrial damage of keratinocytes and fibroblasts. PAHs can also increase ROS discussed above in the skin.

The process of keratinocyte cell proliferation, differentiation and maturation is vulnerable to the many exogenous and endogenous insults that the skin faces on a daily basis. These insults are known to increase the inflammatory response in the epidermis. One consequence of this inflammation is the increased proliferation of keratinocytes followed by poor maturation and differentiation thereof, resulting in a lower quality stratum corneum and thus skin barrier disruption and/or damage. Once the skin barrier has been disrupted or damaged this further enhances the cascade of inflammation and keratinocyte over proliferation, creating a cycle of unhealthy skin traits. The skin barrier is weakened to the attack of pathogens and toxins, increasing the likelihood of skin redness and irritation, pimples and spots and/or causing the skin to appear dull, dry and scaly.

### Summary of the invention

The Applicants have identified that there is a need for a skin treatment regime that achieves a consistent balance for the skin. Currently, individuals may find that treatments for skin conditions themselves cause skin imbalance, e.g. treatments for acne or oiliness can lead to skin dryness, or treatments for dry skin can block pores. Therefore the individual can get caught in a cycle of needing to use varying treatments to each, in turn, compensate for the previous treatment.

In general, it is common for individuals to find that their skin health and appearance fluctuates, e.g. in light of exogenous and/or endogenous insults. Individuals may feel that their skin treatment regime is reactive in nature, trying to respond to the condition in which they find their skin, depending on these exogenous and endogenous insults.

The Applicants have identified a consumer need to provide a proactive skin treatment regime, where the individual can feel in control of their skin condition and encourage a consistent balance in the skin condition.

The Applicants have identified that a kit of cosmetic compositions can be provided which enables an individual to follow a skin treatment regime that can be consistently used on a daily basis to maintain good skin health and appearance.

The Applicants have found that by using the kit of the present invention to follow a daily regime, in which the same cosmetic compositions are applied to the skin each day, fluctuations in skin health and appearance are reduced.

Accordingly, in a first aspect of the invention there is provided a kit of cosmetic compositions, the kit comprising:
(1) a cleansing composition, the cleansing composition comprising: (i) a cosmetically acceptable carrier; (ii) one or more surfactants; (iii) one or more anti-inflammatory agent; and (iv) one or more antioxidant agent selected from polyphenolic antioxidant agents and antioxidant vitamins;
(2) a skin balancing composition, the skin balancing composition comprising: (i) a cosmetically acceptable carrier; (ii) a skin-barrier enhancing agent selected from the group consisting of extracts of *Ophiopogon (e.g. Ophiopogon japonicas),* extracts of Aloe (e.g. *Aloe Vera*), extracts of marshmallow (e.g. *Althaea officianlis*), extracts of Allium (e.g. *Allium cepa*), extracts of tuberose (e.g. *Polianthes tuberosa*), extracts of dandelion, extracts of seaweed (e.g. *Laminaria digitate),* ceramides, and combinations thereof; (iii) one or more antioxidant agent selected from polyphenolic antioxidant agents and antioxidant vitamins; and (iv) one or more sebum control agent selected from the group consisting of sphingosine compounds, *Rosa canina* extract, tea tree extract (e.g. *Melaleuca alternifolia*), witch hazel extract (e.g. *Hamamelis virginiana*), and combinations thereof; and
(3) a daytime protective composition, the daytime protective composition comprising: (i) a cosmetically acceptable carrier; (ii) one or more UV-filter; (iii) one or more antioxidant agent selected from polyphenolic antioxidant agents and antioxidant vitamins; (iv) one or more skin-soothing agent.

In another aspect of the invention, there is provided a method of using the kit according to the invention, in which each of the cosmetic compositions is applied as a topical application on the skin at least once a day.

It is appreciated that the kits of the present invention can be effective in cosmetically treating skin damage as a result of pollution insult or cosmetically preventing the detrimental effects of pollution insult to the skin. Thus, a further aspect of the present invention provides a method of cosmetically treating skin damage as a result of pollution insult, or of cosmetically preventing the detrimental effects of pollution to the skin, said method comprising using the kit according to the invention, in which each of the cosmetic compositions is applied as a topical application on the skin at least once a day.

The cleansing composition can be used to cleanse the skin twice daily (in the morning and in the evening).

The skin balancing composition can be applied after cleansing. It can be applied to the skin once daily (in the morning) or twice daily (in the morning and in the evening).

The daytime protective composition can be applied in the morning, after cleansing and after the skin balancing composition has been applied.

The kit optionally further includes an overnight treatment composition. When an overnight treatment composition is included this can be applied in the evening, after cleansing and after any desired application of skin balancing composition.

The kit optionally further includes a skin improvement composition. This is a product that has characteristics suitable for the individual user's skin type. Therefore it may be an intensive moisture product (for dry skin), or it may be an anti-shine product (for oily or combination skin), or it may be an acne treatment product (for spot-prone skin).

It will be appreciated that this skin improvement composition is optional, because for an individual user with normal skin that is not prone to spots, no such composition is necessarily required.

When a skin improvement composition is included, this can be applied in the morning, after cleansing and after the skin balancing composition has been applied but before the daytime protective composition is applied.

In addition, the skin improvement composition can optionally also be applied in the evening, after cleansing and after the skin balancing composition has been applied but before the overnight treatment composition is applied.

It has been determined that the kit according to the invention provides an individual with a daily skin care regime which allows the skin to be maintained in a more consistent state of good health and appearance.

The combination of the active agents in the cleansing composition, the skin balancing composition, and the daytime protective composition have been found to provide an advantageous daily skincare regime. They target the underlying causes of skin imbalance and help ensure that the skin has a smooth, undamaged top layer that acts as a barrier to keep the skin hydrated and protected, as well as ensuring that oil and moisture levels are balanced. The skin can then respond to exogenous and endogenous insults in a controlled and effective manner.

The active agents in the present kit therefore work together to maintain hydrated, balanced and healthy skin.

### Detailed description of the invention

### Cleansing composition

The kit includes a cleansing composition comprising: (i) a cosmetically acceptable carrier; (ii) one or more surfactants; (iii) one or more anti-inflammatory agent; and (iv) one or more antioxidant agent selected from polyphenolic antioxidant agents and antioxidant vitamins.

The cosmetically acceptable carrier may be water-based, oil-or wax-based, or emulsion-based.

In embodiments where the carrier is emulsion-based, the composition may be in the form of water-in-oil, an oil-in-water, a water-in-oil-in-water or an oil-in-water-in-oil emulsion.

In embodiments where the carrier is water-based, water may be present at a level of about 40% or more, about 45% or more, about 50% or more, about 55% or more, or about 60% or more by weight of the composition.

In embodiments where the carrier is oil-or wax-based, the oil and/or wax may be present at a level of about 15% or more, about 20% or more, about 30% or more, about 25% or more, about 35% or more, or about 40% or more by weight of the composition.

For example, in one embodiment the carrier may be water based and may comprise de-ionized water, purified water, natural spring water, rose water or the like. Mixtures of more than one of these may also be used. In one embodiment de-ionized or purified water is used.

The water based carrier may be 100% water or it may comprise components other than water. These may be components known for use in cosmetic formulations. They may include, but are not limited to, agents such as water-soluble moisturising agents, conditioning agents, anti-microbials, humectants (e.g. glycerin) and/or other water- soluble skin care actives.

In another embodiment, the carrier may be oil or wax based. The oil may be natural oil or synthetic oil, but preferably is natural oil such as a vegetable oil or a nut oil. The oil may be liquid or solid. The wax is preferably a natural wax.

Clearly the oil or wax that is chosen must be able to act as a carrier. Preferably it is a material that can easily be blended at room temperature; thus it may be a liquid at room temperature or a solid that is stirrable at room temperature.

Combinations of one or more oils and/or one or more waxes may be used.

Liquid oils that can be mentioned include avocado oil, Camellia oil, turtle bean oil, macadamia nut oil, corn oil, mink oil, olive oil, Canoga oil, egg yolk oil, sesame seed oil, Persic oil, wheatgerm oil, Camellia sasanqua oil, castor oil, linseed oil, safflower oil, sunflower oil, grapeseed oil, apricot oil, shea oil, sweet almond oil, cotton oil, evening primrose oil, palm oil, perilla oil, hazelnut oil, soybean oil, peanut oil, tea seed oil, kaya oil, rice bran oil, rapeseed oil, alfalfa oil, Chinese tung tree wood oil, Japanese tung tree wood oil, jojoba oil, germ oil, poppyseed oil, pumpkin oil, blackcurrant oil, millet oil, barley oil, quinoa oil, rye oil, candlenut oil, passionflower oil, musk rose oil, triglycerine, glyceryl trioctanoate, and glyceryl triisopalmitate.

Solid oils/fats that can be mentioned include cocoa butter, coconut butter, horse fat, hardened coconut oil, palm oil, beef tallow, mutton tallow, hardened beef tallow, palm kernel oil, lard, Japan wax kernel oil, hardened oil, Japan wax, shea butter, and hardened castor oil.

Waxes that can be mentioned include beeswax, candelilla wax, carnauba wax, lanolin, lanolin acetate, liquid lanolin, sugar cane wax, fatty acid isopropyl lanolin, hexyl laurate, reduced lanolin, jojoba wax, hard lanolin, polyoxyethylene (hereinafter referred to as POE), lanolin alcohol ether, POE lanolin alcohol acetate, lanolin fatty acid polyethylene glycol, and POE hydrogenated lanolin alcohol ether. In one embodiment the carrier is not lanolin based.

Ester oils that can be mentioned include C12-C15 alcohols benzoate, tridecyl salicylate, dibutyl adipate, isopropyl myristate, cetyl octoate, octyldodecyl myristate, isopropyl palmitate, butyl stearate, hexyl laurate, myristyl myristate, decyloleate, hexyldecyl dimethyl octoate, cetyl lactate, myristyl lactate, lanolin acetate, isocetyl stearate, isocetyl isostearate, 12-hydroxy cholesteryl stearate, di-2-ethylhexylic acid ethyleneglycol, dipentaerythritol fatty acid ester, N-alkylglycol monoisostearate, neopentylglycol dicaprate, diisostearyl malate, glyceryl di-2-heptyl undecanate, tri-methylol propane tri-2-ethylhexyl acid, tri-methylol propane triisostearate, pentaerythritol tetra-2-ethylhexyl acid, glyceryl tri-2-ethyl-hexanoate, tri-methylol propane triisostearate, cetyl-2-ethylexanoate, 2-ethylhexyl-palmitate, glycerine trimyristate, glyceride tri-2-heptyl undecatoic acid, methyl ester of castor oil fatty acid, oleate oil, acetoglyceride, palmitate-2-heptyl undecyl, diisopropyl adipate, N-lauroyl-L-glutamic acid-2-octyldodecil ester, di-2-heptylundecyl adipate, di-2-ethylhexyl sebacate, myristate-2-hexyldecyl, palmitate-2-hexyldecyl, adipate-2-hexyldecyl, diisopropyl sebacate, and succinate-2-ethylhexyl.

Higher fatty acids that can be mentioned include lauric acid, myristic acid, palmitic acid, stearic acid, behenic acid, oleic acid, 12-hydroxy-stearic acid, undecylenic acid, lanolin fatty acid, isostearic acid, linoleic acid, linolenic acid, and eicosapentaenoic acid.

Higher alcohols of straight/branched chain that can be mentioned include lauryl alcohol, cetyl alcohol, stearyl alcohol, behenyl alcohol, myristyl alcohol, oleyl alcohol, cetostearyl alcohol, monostearyl glycerine ether (batyl alcohol), 2-decyltetradecinol, lanolin alcohol, cholesterol, phytosterol, hexyldodecanol, isostearyl alcohol, octyldodecanol.

The surfactants in the cleansing composition may be selected from anionic surfactants (e.g. sodium lauryl sulphate, sodium laureth sulphate, ammonium laureth sulphate, disodium laureth sulfosuccinate and sodium C12-15 pareth-12 carboxylate); amphoteric/zwitterionic surfactants (e.g. cocamidopropyl betaine, sodium cocoamphoacetate and cocamidopropyl hydroxysultaine); non-ionic surfactants (e.g. cocamide DEA, cocamide MEA, decyl glucoside, lauryl glucoside); and cationic surfactants (e.g. cetrimonium chloride, behentrimonium chloride and benzalkonium chloride).

In one embodiment the one or more surfactants comprises anionic surfactant. This may be a sulfosuccinate, a carboxylate, a glutamate, a citrate, an isethionate, a glycinate, a sulfolaurate, a taurate, or a combination thereof. In one embodiment, the anionic surfactant is not a sulfate or a sulfonate. The anionic surfactants may be in the form of a salt, for example a sodium salt, a disodium salt and/or a magnesium salt. The anionic surfactant may have a hydrophobic chain of C8-C30, C10-C28, C12-C26, C10-C18, C12-C24, or C12-18.

The anionic surfactant may be selected from the group consisting of Disodium Laureth Sulfosuccinate, Disodium Lauryl Sulfosuccinate, Laureth-7 Citrate, Sodium Cocoyl Glutamate, Sodium Lauroyl Sarcosinate, Sodium Lauryl Glucose Carboxylate, Disodium Cocamido MIPA Sulfosuccinate, Disodium Cocoyl Glutamate, Disodium Oleamido MEA Sulfosuccinate, Disodium PEG-4 Cocamido MIPA-Sulfosuccinate, Disodium PEG-5 Laurylcitrate Sulfosuccinate, Disodium Ricinoleamido MEA-Sulfosuccinate, Disodium Undecylenamido MEA-Sulfosuccinate, Sodium C12-13 Pareth-8 Carboxylate, Sodium C12-15 Pareth-12 Carboxylate, Sodium Laureth-11 Carboxylate, Sodium Laureth-5 Carboxylate, Sodium Lauroyl Glutamate, Sodium Stearoyl Glutamate, Sodium Cocoyl Isethionate, Sodium Isethionate, Sodium Lauroyl Glycinate, Sodium Lauroyl Methyl Isethionate, Sodium Lauryl Glycinate, Sodium Methyl 2-Sulfolaurate, Sodium Methyl Cocoyl Taurate, Sodium Methyl, Lauroyl Taurate, Sodium Methyl Oleoyl Taurate, TEA-Cocoyl Glutamate, and combinations thereof.

The anionic surfactant may be selected from the group consisting of Disodium Laureth Sulfosuccinate, Disodium Lauryl Sulfosuccinate, Laureth-7 Citrate, Sodium Cocoyl Glutamate, Sodium Lauroyl Sarcosinate, Sodium Lauryl Glucose Carboxylate, Disodium Cocamido MIPA Sulfosuccinate, Disodium Cocoyl Glutamate, Disodium Oleamido MEA Sulfosuccinate, Disodium PEG-4 Cocamido MIPA-Sulfosuccinate, Disodium PEG-5 Laurylcitrate Sulfosuccinate, Disodium Ricinoleamido MEA-Sulfosuccinate, Disodium Undecylenamido MEA-Sulfosuccinate, Sodium C12-13 Pareth-8 Carboxylate, Sodium C12-15 Pareth-12 Carboxylate, Sodium Laureth-11 Carboxylate, Sodium Laureth-5 Carboxylate, Sodium Lauroyl Glutamate, Sodium Stearoyl Glutamate, and combinations thereof.

The anionic surfactant may be selected from the group consisting of Disodium Laureth Sulfosuccinate, Disodium Lauryl Sulfosuccinate, Laureth-7 Citrate, Sodium Cocoyl Glutamate, Sodium Lauroyl Sarcosinate, Sodium Lauryl Glucose Carboxylate, and combinations thereof.

In one embodiment the one or more surfactants alternatively or additionally comprises amphoteric surfactant. This may be a betaine, a hydroxysultaine, a diacetate, an acetate, or a combination thereof. The amphoteric surfactants may be in the form of a salt, for example a sodium salt, a disodium salt and/or a magnesium salt. The amphoteric surfactant may have a hydrophobic chain of C8-C30, C10-C28, C12-C26, C10-C18, C12-C24, or C12-18.

The amphoteric surfactant may be selected from the group consisting of Capryl/Capramidopropyl Betaine, Cocamidopropyl Betaine, Sodium Cocoamphoacetate, Babassuamidopropyl Betaine, Cetyl Betaine, Coco-Betaine, Disodium Capryloamphodiacetate, Disodium Cocoamphodiacetate, Disodium Lauroamphodiacetate, Lauramidopropyl Betaine, Lauryl Betaine, Meadowfoamamidopropyl Betaine, Oleamidopropyl Betaine, SheaButteramidopropyl Betaine, Sodium Cocoabutteramphoacetate, Sodium Lauroamphoacetate, Sodium Olivamphoacetate, Soyamidopropyl Betaine, Cocamidopropyl Hydroxysultaine, Lauramidopropyl Hydroxysultaine, Lauryl Hydroxysultaine, and combinations thereof.

The amphoteric surfactant may be selected from the group consisting of Capryl/Capramidopropyl Betaine, Cocamidopropyl Betaine, Sodium Cocoamphoacetate, Babassuamidopropyl Betaine, Cetyl Betaine, Coco-Betaine, Disodium Capryloamphodiacetate, Disodium Cocoamphodiacetate, Disodium Lauroamphodiacetate, Lauramidopropyl Betaine, Lauryl Betaine, Meadowfoamamidopropyl Betaine, Oleamidopropyl Betaine, SheaButteramidopropyl Betaine, Sodium Cocoabutteramphoacetate, Sodium Lauroamphoacetate, Sodium Olivamphoacetate, Soyamidopropyl Betaine, and combinations thereof.

The amphoteric surfactant may be selected from the group consisting of Capryl/Capramidopropyl Betaine, Cocamidopropyl Betaine, Sodium Cocoamphoacetate, and combinations thereof.

In one embodiment the one or more surfactants alternatively or additionally comprises non-ionic surfactant. This may be a glucoside. The non-ionic surfactant may have a hydrophobic chain of C8-C30, C10-C28, C12-C26, C10-C18, C12-C24, or C12-18.

The non-ionic surfactant may be selected from the group consisting of Coco Glucoside Lauryl Glucoside, Caprylyl/Capryl Glucoside, Decyl Glucoside, Polyglyceryl-4 Cocoate, and combinations thereof.

The non-ionic surfactant may be selected from the group consisting of Coco Glucoside Lauryl Glucoside, Caprylyl/Capryl Glucoside, Decyl Glucoside, and combinations thereof.

The non-ionic surfactant may be selected from the group consisting of Coco Glucoside Lauryl Glucoside, and combinations thereof.

The surfactants may be present in an amount of from about 1% to about 50% by weight of the cleansing composition, such as from about 1% to about 45% by weight of the composition, from about 1% to about 40% by weight of the composition, from about 1% to about 35% by weight of the composition, or from about 1 to about 30% by weight of the composition. In one embodiment, surfactant is present in an amount of from about 2% to about 35% by weight of the cleansing composition.

The cleansing composition comprises one or more anti-inflammatory agent. The term "anti-inflammatory agent" is intended to mean an agent which provides an anti-inflammatory benefit as would be understood by a person skilled in the art.

The anti-inflammatory agent may be selected from the group consisting of panthenol, α-bisabolol, betaine, monoammonium glycyrrhizate (MAG), lipochroman, tocopheryl acetate, phytosphingosine, extracts of green tea, extracts of *Sophora flavescens* root, extracts of chamomile (e.g. *Anthemis nobilis),* extracts of *Aloe vera,* extracts of *Echinancea,* extracts of willow bark, extracts of willow herb, extracts of almond, extracts of oats, glycyrrhizic acid, extracts of *Kola,* extracts of red clover, salicylic acid, xymeninic acid, turmeric, and combinations thereof.

The anti-inflammatory agent may be selected from the group consisting of panthenol, α-bisabolol, betaine, monoammonium glycyrrhizate (MAG), lipochroman, tocopheryl acetate, phytosphingosine, extracts of green tea, extracts of *Sophora flavescens* root, extracts of chamomile (e.g. *Anthemis nobilis),* extract of *Aloe vera,* extracts of *Echinancea,* extracts of willow bark, extracts of willow herb, extracts of almond, extracts of oats, glycyrrhizic acid, extracts of *Kola,* extracts of red clover, salicylic acid, and combinations thereof.

The anti-inflammatory agent may be selected from the group consisting of panthenol, α-bisabolol, betaine, monoammonium glycyrrhizate (MAG), lipochroman, tocopheryl acetate, phytosphingosine, extracts of green tea, extracts of willow bark, salicylic acid, and combinations thereof.

The anti-inflammatory agent may be selected from the group consisting of panthenol, α-bisabolol, betaine, monoammonium glycyrrhizate (MAG), lipochroman, tocopheryl acetate, phytosphingosine, extracts of green tea, and combinations thereof.

In one embodiment the cleansing composition comprises two or more anti-inflammatory agents. For example, there may be two or more agents selected from the group consisting of panthenol, α-bisabolol, betaine, monoammonium glycyrrhizate (MAG), lipochroman, tocopheryl acetate, phytosphingosine, extracts of green tea, extracts of willow bark, and salicylic acid.

The anti-inflammatory agent may be present in an amount that produces an inhibitory effect on interleukin-6 (IL-6). For example, the anti-inflammatory agent may produce more than about 40% interleukin-6 inhibition, more than about 50% interleukin-6 inhibition, more than about 60% interleukin-6 inhibition, more than about 70% interleukin-6 inhibition, more than about 75% interleukin-6 inhibition, more than about 80% interleukin-6 inhibition, more than about 90% interleukin-6 inhibition, more than about 99% interleukin-6 inhibition, or 100% interleukin-6 inhibition. In one embodiment, the anti-inflammatory agent, at a concentration of 0.01% by weight of the cosmetic composition, produces more than about 40% interleukin-6 inhibition, more than about 50% interleukin-6 inhibition, more than about 60% interleukin-6 inhibition, more than about 70% interleukin-6 inhibition, more than about 75% interleukin-6 inhibition, more than about 80% interleukin-6 inhibition, more than about 90% interleukin-6 inhibition, more than about 99% interleukin-6 inhibition, or 100% interleukin-6 inhibition. A suitable technique for measuring IL-6 inhibition is described in detail later on in the specification.

The anti-inflammatory agent may be present in an amount of from about 0.0001% to about 20% by weight of the cleansing composition, such as from about 0.0001% to about 15% by weight of the composition, from about 0.0001% to about 10% by weight of the composition, from about 0.0001% to about 5% by weight of the composition, or from about 0.0005 to about 5% by weight of the composition. In one embodiment, the anti-inflammatory agent is present in an amount of from about 0.0005% to about 3% by weight of the cleansing composition.

The cleansing composition comprises one or more antioxidant agent selected from polyphenolic antioxidant agents and antioxidant vitamins.

In one embodiment the cleansing composition comprises one or more polyphenolic antioxidant agents.

The term "polyphenolic" can be defined as a compound which possesses aromatic rings bearing one or more hydroxy substituents, including functional derivatives.

The term "polyphenolic antioxidant agent" is intended to mean a plant, algal or fungal extract, or derivative thereof, comprising one or more species which provide an antioxidant benefit, such as flavonoid species; phenolic acid species; stilbene species; lignin species, or combinations thereof.

In one embodiment the polyphenolic antioxidant agent comprises one or more flavonoid species. Flavonoid species include flavones, flavonols, flavanones, flavanols, anthocyanidins, anthocyanins, proanthocyanidins, flavans, isoflavones and isoflavonoids. Some specific examples of flavonoid species are catechins (catechin, epicatechin, gallocatechin, epigallocatechin, epicatechin gallate, epigallocatechin gallate), quercetin, rutin, hesperidin and genistein.

Plants provide a rich and cheap source of polyphenolic antioxidant agents, and are therefore an efficient source of said agents. Naturally occurring polyphenolic antioxidant agents may therefore be used.

However, the same or similar actives can be also prepared synthetically. The term "polyphenolic antioxidant agent" is therefore intended to cover synthetic polyphenols, such as synthetic analogues of naturally occurring polyphenolic antioxidant agents. Thus chemically synthesized or purified polyphenols and mixtures thereof may be used in place of plant extracts. Polyphenols may be synthesized or extracted from natural sources by any suitable method known to those skilled in the art, particularly using food-grade solvents. Liquid and solid (e.g. granulate or powder form) extracts are suitable.

Extracts (e.g. aqueous or alcoholic) can be obtained from plant parts including but not limited to leaves, raw or cooked whole fruit, berries and vegetables, nuts, the skins of fruit, fruit flesh, fruit rind, peel, pips, cones (e.g. hops), seeds or stones, bark, buds, flowers or parts thereof, including petals and pollen, roots, rhizomes and tubers, and stems. The plant extract may be selected from the group consisting of essential oils, extracts from leaves, extracts from stems, extracts from petals, extracts from seeds, extracts from roots, extracts from pollen, and combinations thereof. In one embodiment, extracts (e.g. lyophilised extracts) from leaves are used.

The polyphenolic antioxidant agent may be selected from the group consisting of extracts of mulberry (e.g. *Morus alba*), ginseng (e.g. *Panax ginseng),* raspberry, oregano (e.g. *Origanum vulgare),* green tea (e.g. green leaves of *Camellia sinensis*), white tea (e.g. *Camellia sinensis*), red tea, Mohani tea, black tea, Oolong tea, yellow tea, jasmine tea, Pu Erh tea, blueberry (e.g. *Vaccinium cyanococcus),* French maritime pine bark (e.g. *Pinus pinaster,* sold under the tradename of Pycnogenol), rosemary (e.g. *Rosmarinus officialis*), grape, including grape seed (e.g. *Vitis vinifera),* fennel (e.g. *Foeniculi fructus), Caragana sinica,* majaoram (e.g. *Origanum majorana),* crocus (e.g. *Crocus sativus),* apple (e.g. *Malus domestica*), coffee, green coffee, cherry (e.g. *Prunus avium*), snow algae (e.g. *Chlamydomonas nivalis),* Emblica (e.g. *Emblica officinalis),* ginkgo (e.g. *Ginkgo biloba*), moringa (e.g. *Moringa oleilera*), ginger, magnolia (e.g. *Magnolioideae virginiana*), French saffron, edelweiss (e.g. *Leontopodium alpinium*), white lotus (e.g. nymphaea alba), turmeric root, marshmallow (e.g. *Althaea officianlis*), burdock (e.g. *Arctium lappa*), bilberry (e.g. *Vaccinium myrtillus),* cranberry (e.g. *Vaccinium oxycoccus),* pomegranate (e.g. *Punica granatum),* sage (e.g. *Salvia officianlis*), thyme (e.g. *Thymus vulgaris*), sunflower (e.g. *Helianthus annus*), wild carrot (e.g. *Daucus carota*), hop (e.g. *Humulus lupulus),* witch hazel (e.g. *Hamamelis),* oak (e.g. *Quercus),* Camellia (e.g. *Theacea*), red clover (e.g. *Tritolium pratense),* flax (e.g. *Linium usitatissiumum),* lemon (e.g. *Citrus limon),* birch (e.g. *Betula),* cornflower (e.g. *Centaurea cyanus),* geranium, polygonum, soy (e.g. *Glycine max*), Sophora (e.g. *Sophora flavescens)* and combinations thereof.

In one embodiment, the polyphenolic antioxidant agent is selected from the group consisting of extracts of: green tea (e.g. green leaves of *Camellia sinensis*), white tea (e.g. *Camellia sinensis*), mulberry (e.g. *Morus alba*), ginseng (e.g. *Panax ginseng*), blueberry (e.g. *Vaccinium cyanococcus),* French maritime pine bark (e.g. *Pinus pinaster,* sold under the tradename of Pycnogenol), rosemary (e.g. *Rosmarinus officialis*), apple (e.g. *Malus domestica*), cherry (e.g. *Prunus avium*), Emblica (e.g. *Pyllanthus emblica),* ginkgo (e.g. *Ginkgo biloba),* moringa (e.g. *Moringa oleilera*), white lotus (e.g. nymphaea alba), marshmallow (e.g. *Althaea officianlis*), bilberry (e.g. *Vaccinium myrtillus),* cranberry (e.g. *Vaccinium oxycoccus),* pomegranate (e.g. *Punica granatum),* thyme (e.g. *Thymus vulgaris*), cornflower (e.g. *Centaurea cyanus),* geranium, polygonum, soy (e.g. *Glycine max*), mulberry (e.g. *Morus alba*), ginseng (e.g. *Panax ginseng),* and Sophora (e.g. *Sophora flavescens,),* burdock (e.g. *Arctium lappa*), and combinations thereof.

In one embodiment, the polyphenolic antioxidant agent is selected from the group consisting of extracts of: green tea (e.g. green leaves of *Camellia sinensis*), Emblica (e.g. *Pyllanthus emblica),* ginkgo (e.g. *Ginkgo biloba),* mulberry (e.g. *Morus alba*), ginseng (e.g. *Panax ginseng),* and Sophora (e.g. *Sophora flavescens)* and combinations thereof.

In one embodiment, the polyphenolic antioxidant agent comprises extracts of green tea. This may optionally be in combination with extracts of: white tea, blueberry, apple, cherry, Emblica, ginkgo, moringa, white lotus, marshmallow, bilberry, cranberry, pomegranate, thyme, cornflower, geranium, polygonum, soy or combinations thereof.

In one embodiment the polyphenolic antioxidant agent has a polyphenol content of 50% or more by dry weight, e.g. 60% or more or 65% or more or 70% or more or 75% or more by dry weight.

The polyphenolic antioxidant agent may be present in an amount of from about 0.0001% to about 20% by weight of the cleansing composition, such as from about 0.0001% to about 15% by weight of the composition, from about 0.0001% to about 10% by weight of the composition, from about 0.0001% to about 5% by weight of the composition, or from about 0.0005 to about 5% by weight of the composition. In one embodiment, the polyphenolic antioxidant agent is present in an amount of from about 0.0005% to about 3% by weight of the cleansing composition.

In one embodiment, the anti-inflammatory agent and the polyphenolic antioxidant agent are the same agent. For example, green tea may be used as an anti-inflammatory agent and as a polyphenolic antioxidant agent in the present invention. In another embodiment, the anti-inflammatory agent and the polyphenolic antioxidant agent are different agents. In one embodiment the skin-soothing agent and the anti-inflammatory agent are the same agent. For example, *Aloe vera* or chamomile may be used as a skin-soothing agent and as an anti-inflammatory agent in the present invention. In another embodiment, the skin-soothing agent and the anti-inflammatory agent are different agents.

In one embodiment the cleansing composition comprises one or more antioxidant vitamins. This may be in addition to, or instead of, polyphenolic antioxidant agents.

The antioxidant vitamin may be selected from the group consisting of vitamin C (ascorbic acid), vitamin E (tocotrienol and/or tocopherol) and beta carotene, derivatives thereof, and combinations thereof.

Derivatives of these antioxidant vitamins include esters and salts. Therefore the antioxidant vitamin may be a vitamin E ester, such as tocopherol acetate, tocopherol n-propionate or tocopherol linoleate, or a vitamin C ester, such as ascorbyl glucoside, ascorbyl palmitate, ascorbyl tetraisopalmitate, magnesium ascorbyl phosphate, sodium ascorbyl phosphate, ascorbyl 2-phosphate 6-palmitate, or 3-O-ethyl ascorbate, or combinations thereof.

In one embodiment the cleansing composition comprises vitamin C or a derivative thereof, e.g. ascorbyl glucoside or ascorbyl palmitate.

The antioxidant vitamin may be present in an amount of from about 0.01% to about 20% by weight of the cleansing composition, such as from about 0.01% to about 15% by weight of the composition, from about 0.01% to about 10% by weight of the composition, from about 0.01% to about 5% by weight of the composition, or from about 0.05 to about 5% by weight of the composition. In one embodiment, the polyphenolic antioxidant agent is present in an amount of from about 0.05% to about 3% by weight of the cleansing composition.

In one embodiment the cleansing composition comprises one or more antioxidant vitamin and one or more polyphenolic antioxidant agents. For example, it may comprise green tea extract and vitamin C or a derivative thereof, e.g. ascorbyl glucoside or ascorbyl palmitate.

The cleansing composition may optionally further comprise one or more skin-soothing agent. The term "skin-soothing agent" is intended to mean an agent which relieves skin discomfort or irritation manifesting as e.g. stinging, burning, tingling, itching, skin tightness, redness, dryness and spots, and which produces a soothing, calming and comforting sensation on the skin.

The skin-soothing agent may be selected from the group consisting of glycerin, butylene glycol, allantoin, shea butter, sodium hyaluronate, bisaccharide gum-1, jojoba oil, isononyl isononanoate, carnauba wax, Aloe Vera, chamomile, comfrey, oatsmeal, coconut oil, honey, cocoa butter, almond oil and combinations thereof

The skin-soothing agent may in one embodiment be selected from the group consisting of glycerin, butylene glycol, allantoin, shea butter, sodium hyaluronate, bisaccharide gum-1, jojoba oil, isononyl isononanoate, carnauba wax, cocoa butter, aloe vera and combinations thereof.

The skin-soothing agent may be selected from the group consisting of glycerin, butylene glycol, shea butter, sodium hyaluronate, bisaccharide gum-1, jojoba oil, isononyl isononanoate, carnauba wax, and combinations thereof.

The skin-soothing agent may be present in an amount that does not produce an inhibitory effect on interleukin-6 (IL-6). For example, the skin-soothing agent may produce less than about 40% interleukin-6 inhibition, less than about 30% interleukin-6 inhibition, less than about 25% interleukin-6 inhibition, less than about 20% interleukin-6 inhibition, less than about 10% interleukin-6 inhibition, or less than about 5% interleukin-6 inhibition. In one embodiment, the skin-soothing agent at a concentration of 0.01% by weight of the cosmetic composition, produces less than about 40% interleukin-6 inhibition, less than about 30% interleukin-6 inhibition, less than about 25% interleukin-6 inhibition, less than about 20% interleukin-6 inhibition, less than about 10% interleukin-6 inhibition, or less than about 5% interleukin-6 inhibition. A suitable technique for measuring IL-6 inhibition is described in detail later on in the specification.

The skin-soothing agent may be present in an amount of from about 0.01% to about 50% by weight of the composition, such as about 0.05% to about 45% by weight of the composition, about 0.1% to about 40% by weight of the composition, about 0.5% to about 35% by weight of the composition, or about 1 to about 35% by weight of the composition. In one embodiment, the skin-soothing agent is present in an amount of from about 0.5% to about 30% by weight of the composition.

In one embodiment, the cleansing composition is in the form of a gel, cream or lotion.

### Skin balancing composition

The kit includes a skin balancing composition, the skin balancing composition comprising (i) a cosmetically acceptable carrier; (ii) a skin-barrier enhancing agent selected from the group consisting of extracts of *Ophiopogon (e.g. Ophiopogon japonicas),* extracts of Aloe (e.g. *Aloe Vera*), extracts of marshmallow (e.g. *Althaea officianlis*), extracts of Allium (e.g. *Allium cepa*), extracts of tuberose (e.g. *Polianthes tuberosa*), extracts of dandelion, extracts of seaweed (e.g. *Laminaria digitate),* ceramides, and combinations thereof; (iii) one or more antioxidant agent selected from polyphenolic antioxidant agents and antioxidant vitamins; (iv) one or more sebum control agent selected from the group consisting of sphingosine compounds, *Rosa canina* extract, tea tree extract (e.g. *Melaleuca alternifolia*), witch hazel extract (e.g. *Hamamelis virginiana*), and combinations thereof.

The cosmetically acceptable carrier may be as described above in relation to the cleansing composition.

The skin-balancing composition includes a skin-barrier enhancing agent selected from the group consisting of extracts of *Ophiopogon (e.g. Ophiopogon japonicas),* extracts of Aloe (e.g. *Aloe Vera*), extracts of marshmallow (e.g. *Althaea officianlis*), extracts of Allium (e.g. *Allium cepa*), extracts of tuberose (e.g. *Polianthes tuberosa*), extracts of dandelion, extracts of seaweed (e.g. *Laminaria digitate),* ceramides, and combinations thereof.

In one embodiment, the skin-barrier enhancing agent is *Ophiopogon japonicas* extract.

The *Ophiopogon japonicas* extract (e.g. aqueous or alcoholic) may be from leaves, raw or cooked whole fruit, the skins of fruit, fruit flesh, buds, flowers or parts thereof, including petals and pollen, roots, tubers, and stems. In one embodiment extracts from roots/tubers are used.

The extract may be obtained by a method including the steps of:
- solubilization of *Ophiopogon japonicus* tuber powders in water,
- at least one enzymatic hydrolysis,
- separation of soluble and insoluble phases, and
- enzymatic inactivation by heat treatment.

Enzymatic inactivation can be followed by one or more filtration and/or concentration stages. Preferably, the enzymatic inactivation is followed by at least one filtration stage and one sterilizing filtration stage.

The enzymes that are used are carbohydrases. They can be purified enzymes, mono-constituent enzymes, or else a mixture of different enzymes.

The active ingredient can be obtained in liquid form or in powder form by atomization or by freeze-drying.

In one embodiment the extract comprises 50% or more, such as 60% or more, fructosans by weight, relative to the total weight of the dry extract.

The one or more antioxidant agent selected from polyphenolic antioxidant agents and antioxidant vitamins may be as described above in relation to the cleansing composition.

The skin balancing composition includes one or more sebum control agent selected from the group consisting of sphingosine compounds, *Rosa canina* extract, tea tree extract (e.g. *Melaleuca alternifolia*), witch hazel extract (e.g. *Hamamelis virginiana*), and combinations thereof.

In one embodiment, the sebum control agent is selected from sphingosine compounds, *Rosa canina* extract, and combinations thereof.

In one embodiment the skin balancing composition includes *Rosa canina* extract.

The *Rosa canina* extract (e.g. aqueous or alcoholic) may be from leaves, raw or cooked whole fruit, the skins of fruit, fruit flesh, fruit rind, seeds, buds, flowers or parts thereof, including petals and pollen, roots, and stems. In one embodiment, extracts (e.g. lyophilised extracts) from leaves or from fruit are used. In one embodiment, extracts from fruit are used.

In one embodiment the skin balancing composition includes one or more sphingosine compound. This may be in addition to, or instead of, *Rosa canina* extract.

The sphingosine compound may be a compound of Formula (I), or a salt or derivative thereof: 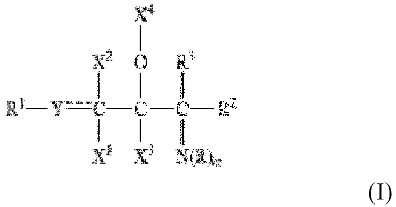 wherein:
R¹ is a linear, branched or cyclic, saturated or unsaturated C4-30 hydrocarbon group, which may optionally be substituted by a hydroxyl, carbonyl or amino group,
Y is any one of a methylene group (CH₂), a methine group (CH) and an oxygen atom,
X¹, X² and X³ are each independently a hydrogen atom, a hydroxyl group or an acetoxy group,
X⁴ is a hydrogen atom, an acetyl group, a glyceryl group or a substituent forming an oxo group together with the adjacent oxygen atom.
R² and R³ are each independently a hydrogen atom, a hydroxyl group, a hydroxymethyl group or an acetoxymethyl group.
a is an integer which is 2 or 3, and
each R is each independently a hydrogen atom, or an amidino group, or a linear or branched, saturated or unsaturated hydrocarbon group having 1 to 8 carbon atoms in total and optionally having a substituent selected from hydroxyl, hydroxyalkoxy, alkoxy and acetoxy groups.

The derivative may be an N-acyl, O-acyl or N-alkyl derivative. In one embodiment it is an N-acyl or N-alkyl derivative.

The salt may, for example, be a hydrochloride, phosphate, sulphate, nitrate, chloride, or other soluble salt.

In one embodiment R¹ is a linear, branched or cyclic, saturated or unsaturated C7-22 (e.g. C8-C20 or C9-C20) hydrocarbon group which may be substituted by a hydroxyl group. In one embodiment R¹ is a linear or branched C10-20 (e.g. C12-C18) alkyl group, or a linear or branched C10-20 (e.g. C12-C18) alkyl group having, at a terminal thereof on the Y side, a hydroxyl group. When it is a branched alkyl group, it preferably is a methyl-branched alkyl chain. Examples include tridecyl, tetradecyl, pentadecyl, hexadecyl, 1-hydroxytridecyl, 1-hydroxypentadecyl, isohexadecyl and isostearyl groups.

In one embodiment, at most one (i.e. none or exactly one) of the X¹, X² and X³ groups is a hydroxyl group, the remainder of the X¹, X² and X³ groups are each a hydrogen atom, and X⁴ is a hydrogen atom.

When Y is a methine group, either X¹ or X² is a hydrogen atom and the other one does not exist.

When X⁴ forms an oxo group, X³ does not exist.

In one embodiment, one of R² and R³ is a hydrogen atom. In one embodiment, one of R² and R³ is a hydroxymethyl group. In one embodiment, one of R² and R³ is a hydrogen atom and the other is a hydroxymethyl group.

As noted above, the R groups may be an optionally substituted hydrocarbon group. In one embodiment, the hydroxyalkoxy group which may be a substituent for the hydrocarbon group is a linear or branched C1-7 hydroxyalkoxy group and the alkoxy group which may be a substituent for the hydrocarbon group is a linear or branched C1-7 alkoxy group.

In one embodiment, the R groups are independently selected from a hydrogen atom; linear or branched alkyl groups such as methyl, ethyl, propyl, 2-ethylhexyl and isopropyl; alkenyl groups such as vinyl and allyl; amidino groups; and hydrocarbon groups having 1 to 8 carbon atoms in total and having 1 to 6 substituents selected from hydroxyl group, hydroxyalkoxy group and alkoxy groups, such as hydroxymethyl, 2-hydroxyethyl, 1,1-dimethyl-2-hydroxyethyl, 2-hydroxypropyl, 2,3-dihydroxypropyl, 2-hydroxy-3-methoxypropyl, 2,3,4,5,6-pentahydroxyhexyl, 1,1-bis(hydroxymethyl)ethyl, 2-(2-hydroxyethoxy)ethyl, 2-methoxyethyl, 1-methyl-2-hydroxyethyl, 3-hydroxypropyl, 3-methoxypropyl, and 1,1-bis(hydroxymethyl)-2-hydroxyethyl.

In one embodiment, the R groups are independently selected from a hydrogen atom, a methyl group, and an alkyl group which may be substituted by 1 to 3 substituents selected from hydroxyl group and hydroxyalkoxy groups, such as 2-hydroxyethyl, 1,1-dimethyl-2-hydroxyethyl, 1,1-bis(hydroxymethyl)ethyl, 2-(2-hydroxyethoxy)ethyl.

In one embodiment, the R groups are each hydrogen.

The sphingosine compound may be selected from the group consisting of: sphingosine; phytosphingosine; dihydrosphingosine; derivatives thereof (including salts thereof); and combinations thereof. For example, the sphingosine compound may be an N-acyl, O-acyl or N-alkyl derivative. The sphingosine compound may be a salt, for example a hydrochloride, a phosphate, a nitrate, a sulphate, or combinations thereof. The sphingosine compound may be phytosphingosine hydrochloride.

The sphingosine compound may be a ceramide, for example ceramide 1, ceramide 2, ceramide 3 and/or ceramide 6. However, in another embodiment, the sphingosine compound is not a ceramide.

For the sphingosine compound, the natural (D(+) form) optically active derivatives, the natural (L(-) form) optically active derivatives, or a mixture thereof, may be used. The relative configuration of the sphingosine compounds may therefore be any one of the natural form, an unnatural form, or a mixture thereof.

In one embodiment, the sphingosine compound is selected from the group consisting of 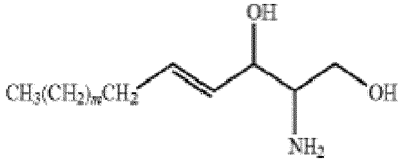 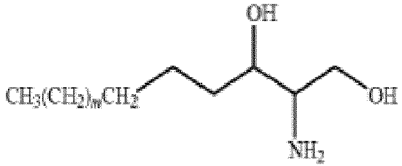 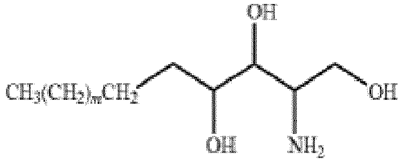 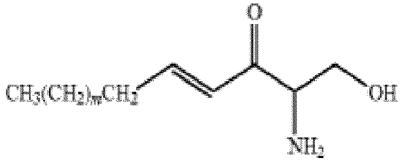 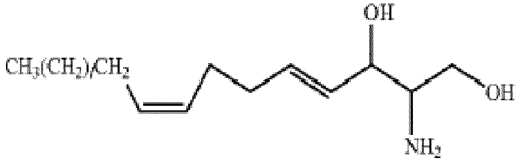 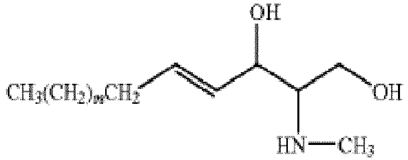 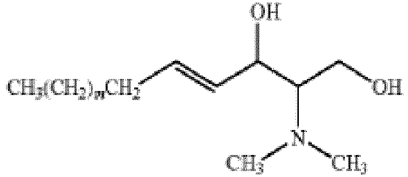 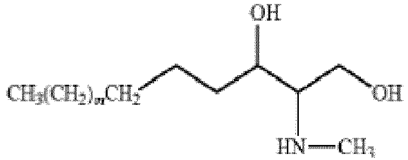 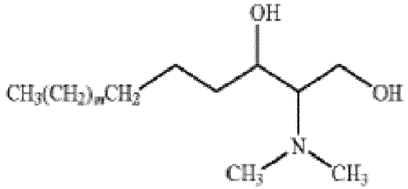 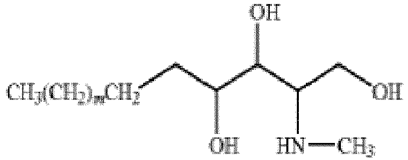 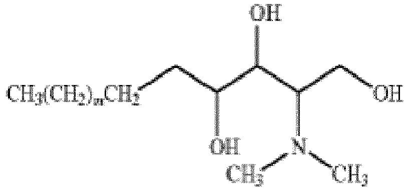 wherein m is an integer from 5 to 17 and 1 is an integer from 1 to 13.

In one embodiment, the sphingosine compound is selected from the group consisting of: natural sphingosine, dihydrosphingosine, phytosphingosine, sphingadienine, dehydrosphingosine, and dehydrophytosphingosine, salts thereof (e.g. phytosphingosine hydrochloride), and N-alkyl derivatives (e.g., N-methyl derivatives) thereof.

In one embodiment, the sphingosine compound is selected from the group consisting of: dihydrosphingosine (sphinganine), phytosphingosine, salicyloyl phytosphingosine (phytosphingosine SLC), phytosphingosine hydrochloride (phytosphingosine HCl), sphingonine hydroxysphingosine, and sphingosine-1-phosphate.

In one embodiment, the sphingosine compound comprises dihydrosphingosine.

The sphingosine compound may be natural or may be synthetic. Thus it may be obtained from any suitable source, for example from a natural source or from a chemical synthesis process. The sphingosine compound may be obtained from an animal source, a plant source, a yeast source, or combinations thereof. In one embodiment, the sphingosine compound is obtained from a plant source.

The sphingosine compound may be present in an amount of from about 0.001% to about 10% by weight of the composition, e.g. from about 0.01% to about 5% by weight of the composition, about 0.01% to about 3% by weight of the composition, about 0.01% to about 2% by weight of the composition, or about 0.01 to about 1% by weight of the composition. In one embodiment, sphingosine compound is present in an amount of from about 0.01% to about 0.5% by weight of the composition.

In one embodiment, the skin balancing composition is in the form of a serum.

The serum is suitably water-based. Water may be present at a level of about 50% or more, about 55% or more, about 60% or more, or about 65% or more; it may be that there is from 50 to 85% water by weight of the composition.

### Daytime protective composition

The kit includes a daytime protective composition, the daytime protective composition comprising (i) a cosmetically acceptable carrier; (ii) one or more UV-filter; (iii) one or more antioxidant agent selected from polyphenolic antioxidant agents and antioxidant vitamins; (iv) one or more skin-soothing agent.

The cosmetically acceptable carrier may be as described above in relation to the cleansing composition.

The UV-filter may be selected from: inorganic sunscreen agents and organic sunscreen agents.

Inorganic sunscreen agents include microfine titanium dioxide, microfine zinc oxide, iron oxides, talcs and boron nitride.

Organic sunscreen agents include p-aminobenzoic acids, esters and derivatives thereof (e.g. 2-ethylhexyl p-dimethyl-aminobenzoate), esters of benzoic acid (e.g. 2-hydroxy- 2-ethylhexyl ester benzoic acid, known as octyl salicylate), methoxycinnamate esters (e.g., 2-ethylhexyl p-methoxycinnamate, known as octinoxate, 2-ethoxyethyl p-methoxycinnamate or α,β-di-(p-methoxycinnamoyl)-α'-(2ethylhexanoyl)-glycerin), benzophenones (e.g. oxybenzone), dibenzoylmethanes (e.g. 4-(tert-butyl)-4'-methoxydibenzoylmethane, known as Avobenzone), 2-phenylbenzimidazole-5 sulfonic acid and salts thereof, alkyl-β,β-diphenylacrylates (e.g. alkyl α-cyano-β,β-diphenylacrylates, such as octocrylene), triazines (such as 2,4,6-trianilino-(p-carbo-2-ethyl-hexyl-1-oxy)-1,3,5 triazine), methylene bis benzotriazoyl tetramethylbutylphenol and/or camphor derivatives (such as methylbenzylidene camphor and camphor benzalkonium methosulfate, diethylamino hydroxybenzoyl hexyl benzoate).

The one or more UV-filters may be present in an amount of from about 0.1 to about 35% by weight of the composition, such as from about 0.5% to about 30% by weight of the composition, about 0.5% to about 25% by weight of the composition, about 1% to about 25% by weight of the composition, or about 1 to about 20% by weight of the composition. In one embodiment, UV-filter is present in an amount of from about 2% to about 20% by weight of the composition.

The antioxidant agent selected from polyphenolic antioxidant agents and antioxidant vitamins may be as described above in relation to the cleansing composition.

The skin-soothing agent may be as described above in relation to the cleansing composition.

In one embodiment, the daytime protective composition is in the form of a cream or lotion.

The composition is suitably water-based. It may be an oil-in-water emulsion. Water may be present in an amount of about 40% to about 90% by weight of the composition, such as from about 50% to about 85% or about 55% to about 75% by weight of the composition.

### Optional overnight treatment composition

As noted above, the kit may include an overnight treatment composition, the overnight treatment composition comprising (i) a cosmetically acceptable carrier; (ii) one or more polyphenolic antioxidant agent; (iii) one or more antioxidant vitamin; (iv) one or more skin-soothing agent.

The cosmetically acceptable carrier may be as described above in relation to the cleansing composition.

The polyphenolic antioxidant agent may be as described above in relation to the cleansing composition.

The antioxidant vitamin may be as described above in relation to the cleansing composition.

The skin-soothing agent may be as described above in relation to the cleansing composition.

In one embodiment, the overnight treatment composition is in the form of a cream or lotion.

The composition is suitably water-based. It may be an oil-in-water emulsion. Water may be present in an amount of about 55% to about 95% by weight of the composition, such as from about 60% to about 90% or about 65% to about 90% by weight of the composition.

### Optional skin improvement composition

As noted above, the kit may include a skin improvement composition. This is a product that has characteristics suitable for the individual user's skin type. Therefore it may be an intensive moisture product (for dry skin), or it may be an anti-shine product (for oily or combination skin), or it may be an acne treatment product (for spot-prone skin).

When the skin improvement composition is an intensive moisture product, it may comprise: (i) a cosmetically acceptable carrier; (ii) a skin-barrier enhancing agent selected from the group consisting of extracts of *Ophiopogon (e.g. Ophiopogon japonicas),* extracts of Aloe (e.g. *Aloe Vera*), extracts of marshmallow (e.g. *Althaea officianlis*), extracts of Allium (e.g. *Allium cepa*), extracts of tuberose (e.g. *Polianthes tuberosa*), extracts of dandelion, extracts of seaweed (e.g. *Laminaria digitate),* ceramides, and combinations thereof; (iii) one or more antioxidant agent selected from polyphenolic antioxidant agents and antioxidant vitamins; (iv) one or more skin-soothing agent.

The cosmetically acceptable carrier may be as described above in relation to the cleansing composition.

The skin-barrier enhancing agent may be described as above in relation to the skin-balancing composition.

In one embodiment, the skin-barrier enhancing agent may be *Ophiopogon japonicas* extract. The *Ophiopogon japonicas* extract may be as described above in relation to the skin balancing composition.

The antioxidant agent may be as described above in relation to the cleansing composition.

In one embodiment the intensive moisture product comprises one or more antioxidant vitamin and one or more polyphenolic antioxidant agents. For example, it may comprise green tea extract and vitamin C or a derivative thereof, e.g. ascorbyl glucoside or ascorbyl palmitate.

The skin-soothing agent may be as described above in relation to the cleansing composition.

In one embodiment, the intensive moisture product is in the form of a cream or lotion.

The composition is suitably water-based. It may be an oil-in-water emulsion. Water may be present in an amount of about 55% to about 95% by weight of the composition, such as from about 60% to about 90% or about 65% to about 90% by weight of the composition.

When the skin improvement composition is an anti-shine product, it may comprise: (i) a cosmetically acceptable carrier; (ii) one or more silicone agent; (iii) one or more antioxidant agent selected from polyphenolic antioxidant agents and antioxidant vitamins; (iv) one or more skin-soothing agent.

The cosmetically acceptable carrier may be as described above in relation to the cleansing composition.

The silicone agent may be a silicone elastomer. Such materials may be formed by reacting a methyl hydrogen polysiloxane with a cross-linking agent (e.g. a vinyl-containing compound) in the presence of a silicone solvent and a platinum catalyst.

The silicone agent may comprise an organopolysiloxane elastomer dispersed in a silicone-compatible vehicle. In one embodiment the silicone agent may comprise a cross-linked gel dispersed in silicone or hydrocarbon vehicle. Examples of vehicles are dimethicone, cyclopentasiloxane, isododecane, isohexadecane, and isononyl isononanoate.

In one embodiment, the silicone agent comprises linear dimethyl polysiloxane.

The silicone agent may be present in an amount of from about 0.05% to about 10% by weight of the composition, e.g. from about 0.1% to about 5% by weight of the composition, about 0.1% to about 4% by weight of the composition, about 0.1% to about 3% by weight of the composition, or about 0.1 to about 2% by weight of the composition. In one embodiment, silicone agent is present in an amount of from about 0.5% to about 2% by weight of the composition.

The antioxidant agent may be as described above in relation to the cleansing composition.

In one embodiment the intensive moisture product comprises one or more antioxidant vitamin and one or more polyphenolic antioxidant agents. For example, it may comprise green tea extract and vitamin C or a derivative thereof, e.g. ascorbyl glucoside or ascorbyl palmitate.

The skin-soothing agent may be as described above in relation to the cleansing composition.

In one embodiment, the anti-shine product is in the form of a cream or lotion.

The composition is suitably water-based. It may be an oil-in-water emulsion. Water may be present in an amount of about 55% to about 95% by weight of the composition, such as from about 60% to about 90% or about 65% to about 90% by weight of the composition.

When the skin improvement composition is an acne treatment product, it may comprise: (i) a cosmetically acceptable carrier; (ii) one or more salicylic acid compound; (iii) one or more antioxidant agent selected from polyphenolic antioxidant agents and antioxidant vitamins; (iv) one or more anti-inflammatory agent.

The cosmetically acceptable carrier may be as described above in relation to the cleansing composition.

The salicylic acid compound may be salicylic acid (i.e. 2-hydroxybenzoic acid) or a derivative thereof. The derivative may be in the form of a salt of salicylic acid such as a metal salt or ammonium salt. For example, a sodium, potassium, ammonium, triethanolammonium, glucammonium, copper, titanium, or zinc salt of salicylic acid. Other derivatives of salicylic acid include benzyl salicylate, betaine salicylate, thiosalicylic acid and salicylic acid esters, such as alkyl esters of C1-C20. In one embodiment, the salicylic acid compound is salicylic acid.

The salicylic acid compound may be present in an amount of from about 0.01% to about 10% by weight of the composition, e.g. in an amount of about 0.01% to about 7% by weight of the composition, about 0.05% to about 6% by weight of the composition, about 0.05% to about 5% by weight of the composition, about 0.1 to about 4% by weight of the composition. In one embodiment, the salicylic acid compound is present in an amount of about 0.5% to about 3% by weight of the composition.

The antioxidant agent may be as described above in relation to the cleansing composition.

The anti-inflammatory agent may be as described above in relation to the cleansing composition.

In one embodiment, the acne treatment product is in the form of a cream or lotion.

The composition is suitably water-based. In one embodiment it is based on a carrier that comprises water and alcohol (denatured ethanol). Water may be present in an amount of about 50% to about 85% by weight of the composition, such as from about 55% to about 80% or about 55% to about 75% by weight of the composition. Alcohol may be present in an amount of about 10% to about 40% by weight of the composition, such as from about 15% to about 35% or about 20% to about 35% by weight of the composition.

### General

The following comments apply independently to each of the compositions included in the kit, unless stated otherwise.

The composition may be provided in any form suitable for topical application to the skin. The composition may be delivered and/or applied to the skin via any of the conventional formulations known to those skilled in the art. Typical formulation types of the present invention are creams, lotions, milks, gels, serums, foams, and ointments.

The composition may be water-based, oil-based, microemulsion-based or emulsion-based. In embodiments where the cosmetic composition is emulsion-based, the composition may be in the form of a water-in-oil, an oil-in-water, a water-in-oil-in-water or an oil-in-water-in-oil emulsion. Preferably, the cosmetic composition is in the form of an oil-in-water emulsion.

In embodiments where the composition is in the form of a water-in-oil emulsion, water may be present at a level of about 20% to about 60% by weight of the composition, about 20% to about 50% by weight of the composition, or about 35% to about 45% by weight of the composition. In one embodiment where the cosmetic composition is in the form of a water-in-oil emulsion, water is present at a level of about 35% to about 45% by weight of the composition.

In embodiments where the composition is in the form of an oil-in-water emulsion, water may be present in an amount of about 40% to about 90% by weight of the composition or about 60% to about 95% by weight of the composition. In one embodiment where the cosmetic composition is in the form of an oil-in-water emulsion, water is present at a level of about 60% to about 95% by weight of the composition.

The composition will generally further comprise other ingredients or excipients which will be well known to those skilled in the art.

For example, the composition may further comprise one or more humectants, including but not limited to glycerin, propylene glycol, propanediol, butylene glycol, hexylene glycol, hexanediol, dipropylene glycol, polyethylene glycol, sorbitol, urea, xylitol, lactitol, fructose, glucose, mannose, xylose, honey, pyrrolidone, and carboxylic acid, pentylene glycol, sodium hyaluronate and/or salts thereof. When present, the one or more humectants may be present in an amount of about 0.01% to about 20% by weight of the composition, about 0.1% to about 10%, or about 0.5% to about 7% by weight of the composition.

The composition may further comprise one or more emollients, including but not limited to PPG-15 stearyl ether, ethylhexyl stearate, cetyl dimethicone, octyldodecanol, PPG-20 methyl glucose ether, isopropyl myristate, isopropyl paltimate, isopropyl laurate, isodecyl laurate, isodecyl neopentanoate, isohexadecane, pentaerythrityl tetraisostearate, caprylic/capric triglyceride, canola oil, sunflower oil (*Helianthus annus*), olive oil (*Olea europea*), cottonseed oil (*Gossypium herbaceum*), jojoba oil (*Simmondsia chinensis*), shea butter (*Butyrospermum parkii),* cocoa butter *(Theobroma cacao*), cupuacu butter (*Theobroma grandiflorum*), avocado oil (*Persea gratissima*), liquid paraffin, dimethicone, phenyl trimethicone, cyclopentasiloxane, dimethiconol, sodium hyaluronate, bisaccharide gum, isopropyl isononoate, Carnauba wax, and/or petrolatum. When present, the one or more emollients may be present in an amount of about 0.01% to about 20% by weight of the composition, about 0.1% to about 10%, or about 0.5% to about 7% by weight of the composition.

The composition may further comprise one or more emulsifiers, including but not limited to steareth-2, steareth-21, steareth-10, ceteareth-5, ceteareth-20, cetearyl glucoside, oleth-10, glyceryl stearate, polyglycerol-3 oleate, polyglyceryl-3 methylglucose distearate, sodium stearate, PEG-12 oleate, PEG-2 stearate, PEG-12 stearate, PEG-100 stearate, cetyl alcohol, cetearyl alcohol, potassium cetyl phosphate, cetearyl olivate, sorbitan olivate, PEG-80 sorbitan, sorbitan oleate, and/or sorbitan palmitate. In one embodiment, the cosmetic composition of the invention does not comprise sulphates as emulsifiers. In embodiments where one or more emulsifiers are present, the one or more emulsifiers may be present in an amount of about 0.1% to about 10% by weight of the composition, about 0.25% to about 7.5% by weight of the composition, or about 0.5% to about 5% by weight of the composition. In one embodiment where one or more emulsifiers are present in the cosmetic composition, the one or more emulsifiers are present in an amount of about 0.5% to about 5% by weight of the composition.

As noted above, the cleansing composition includes surfactant. The other compositions in the kit may optionally comprise one or more surfactants, including but not limited to, anionic surfactants (e.g. sodium lauryl sulphate, sodium laureth sulphate, ammonium laureth sulphate, disodium laureth sulfosuccinate and sodium C12-15 pareth-12 carboxylate), amphoteric/zwitterionic surfactants (e.g. cocamidopropyl betaine, sodium cocoamphoacetate and cocamidopropyl hydroxysultaine), non-ionic surfactants (e.g. cocamide DEA, cocamide MEA, decyl glucoside, lauryl glucoside), and cationic surfactants (e.g. cetrimonium chloride, behentrimonium chloride and benzalkonium chloride). In one embodiment, the composition does not comprise sulphates as surfactants. In embodiments where one or more surfactants are present, the one or more surfactants may be present in an amount of from about 0.1% to about 10% by weight of the composition, about 0.25% to about 7.5% by weight of the composition, or about 0.5% to about 5% by weight of the composition. In one embodiment where one or more surfactants are present in any of the cosmetic compositions that are not the cleansing composition, the one or more surfactants are present in an amount of from about 0.5% to about 3% by weight of the composition.

The composition may further comprise one or more preservatives, including but not limited to, 2-bromo-2nitropropane-1,3-diol (bronopol, commercially available under the trade name Myacide RTM), benzyl alcohol, benzoic acid, sodium benzoate, diazolidinyl urea, imidazolidinyl urea, methyl paraben, phenoxyethanol, ethyl paraben, propyl paraben, sodium methyl paraben, sodium dehydroacetate, dehydroacetic acid, polyhexamethylenebiguanide hydrochloride, isothiazolone, chlorhexidine digluconate, chlorphensin and/or sodium propyl paraben. In one embodiment, the composition does not comprise parabens. In embodiments where one or more preservatives are present, the one or more preservatives may be present in an amount of about 0.001% to about 10% by weight of the composition, about 0.01% to about 8% by weight of the composition, or about 0.1% to about 5% by weight of the composition. In one embodiment where one or more preservatives are present in the cosmetic composition, the one or more preservatives are present in an amount of about 0.05% to about 8% by weight of the composition.

The composition may further comprise one or more chelating agents or sequestering agents, including but not limited to, ethylenediamine tetraacetic acid (EDTA) and salts thereof (e.g. dipotassium EDTA, disodium EDTA or tetrasodium EDTA), sodium phytate, trisodium ethylene diamine disuccinate, and/or tetrasodium glutamate diacetate. In embodiments where one or more chelating agents are present, the one or more chelating agents may be present in an amount of about 0.001% to about 10% by weight of the composition, about 0.01% to about 8% by weight of the composition, or about 0.1% to about 5% by weight of the composition. In one embodiment where one or more chelating agents are present, the one or more chelating agents are present in an amount of about 0.05% to about 8% by weight of the composition.

The composition may further comprise one or more vitamins. This is in addition to any antioxidant vitamins that are included. For example, the composition may further comprise vitamin B, vitamin B1 to vitamin B12, vitamin D, vitamin K, vitamin H, derivatives thereof, provitamins thereof (e.g. provitamin B5 (panthenol)), or combinations thereof. In embodiments where one or more vitamins are present, the one or more vitamins may be present in an amount of about 0.0001% to about 50% by weight of the composition, about 0.001% to about 10% by weight of the composition, about 0.01% to about 8% by weight of the composition, or about 0.1% to about 5% by weight of the composition. In one embodiment where one or more vitamins are present, the one or more vitamins are present in an amount of about 0.1% to about 5% by weight of the composition.

The composition may further comprise one or more pH adjusting agents, including but not limited to potassium hydroxide, sodium hydroxide, aminomethyl propanol, sodium citrate and/or triethanolamine. The composition may have a pH from about 3 to about 10, e.g. from about 4 to about 8, or from about 5 to about 7. In embodiments where one or more pH adjusting agents are present, the one or more pH adjusting agents may be present in an amount of from about 0.01 to about 10% by weight of the composition.

The composition may further comprise one or more thickeners or gelling agents. For example, when the composition is in the form of a gel, the composition may comprise one or more thickeners or gelling agents. Examples of thickeners/gelling agents that can be used in the present invention include, but are not limited to, acrylic acid polymers (e.g. available commercially under the trade name Carbopol or Ultrez (Lubrizol), modified celluloses (e.g. hydroxyethylcellulose available commercially under the trade name Natrosol from Hercules) hydroxypropylmethyl cellulose, amine oxides, block polymers of ethylene oxide and propylene oxide (e.g. those available from BASF Wyandotte under the trade name "Pluronic"), PVM, MA, decadiene crosspolymer (e.g. available under the trade name Stabilez 60), ethoxylated fatty alcohols, salt (e.g. magnesium chloride, sodium chloride), Aristoflex AVC, phthalic acid amide, xanthan gum, sodium polyacrylate, polyvinyl alcohols, fatty alcohols, and/or alkyl galactmanans (e.g. available under the trade name N-Hance from Hercules. In embodiments where one or more thickeners/gelling agents are present, the one or more thickeners/gelling agents may be present in an amount of about 0.01 to about 10% by weight of the composition.

The composition may further comprise one or more perfumes and/or colourings.

In the present application, the term "about" may encompass ±10%, such as ±5%, e.g. ±2% or ±1%.

### Specific compositions

In one embodiment, the kit of the invention comprises:
1) a cleansing composition, the cleansing composition comprising: (i) a cosmetically acceptable carrier; (ii) one or more surfactants; (iii) one or more anti-inflammatory agent; (iv) one or more antioxidant agent selected from polyphenolic antioxidant agents and antioxidant vitamins; (v) one or more skin-soothing agent;
2) a skin balancing composition, the skin balancing composition comprising: (i) a cosmetically acceptable carrier; (ii) *Ophiopogon japonicas* extract; (iii) one or more antioxidant agent selected from polyphenolic antioxidant agents and antioxidant vitamins; (iv) one or more sebum control agent selected from *Rosa canina* extract and sphingosine compounds;
3) a daytime protective composition, the daytime protective composition comprising: (i) a cosmetically acceptable carrier; (ii) one or more UV-filter; (iii) one or more antioxidant agent selected from polyphenolic antioxidant agents and antioxidant vitamins; (iv) one or more skin-soothing agent;
4) an overnight treatment composition, the overnight treatment composition comprising: (i) a cosmetically acceptable carrier; (ii) one or more polyphenolic antioxidant agent; (iii) one or more antioxidant vitamin; (iv) one or more skin-soothing agent,
wherein in (1), the anti-inflammatory agent is selected from the group consisting of panthenol, α-bisabolol, betaine, monoammonium glycyrrhizate (MAG), lipochroman, tocopheryl acetate, phytosphingosine, extracts of green tea, extracts of *Sophora flavescens* root, extracts of chamomile (e.g. *Anthemis nobilis),* extracts of *Aloe vera,* extracts of *Echinacea,* extracts of willow bark, extracts of willow herb, extracts of almond, extracts of oats, glycyrrhizic acid, extracts of *Kola,* extracts of red clover, salicylic acid, xymeninic acid, turmeric, and combinations thereof.

In one embodiment, the kit of the invention comprises:
1) a cleansing composition, the cleansing composition comprising: (i) a cosmetically acceptable carrier; (ii) one or more surfactants; (iii) one or more anti-inflammatory agent; (iv) one or more antioxidant agent selected from polyphenolic antioxidant agents and antioxidant vitamins; (v) one or more skin-soothing agent;
2) a skin balancing composition, the skin balancing composition comprising: (i) a cosmetically acceptable carrier; (ii) *Ophiopogon japonicas* extract; (iii) one or more antioxidant agent selected from polyphenolic antioxidant agents and antioxidant vitamins; (iv) one or more sebum control agent selected from *Rosa canina* extract and sphingosine compounds;
3) a daytime protective composition, the daytime protective composition comprising: (i) a cosmetically acceptable carrier; (ii) one or more UV-filter; (iii) one or more antioxidant agent selected from polyphenolic antioxidant agents and antioxidant vitamins; (iv) one or more skin-soothing agent;
4) an overnight treatment composition, the overnight treatment composition comprising: (i) a cosmetically acceptable carrier; (ii) one or more polyphenolic antioxidant agent; (iii) one or more antioxidant vitamin; (iv) one or more skin-soothing agent,
wherein in (1), the anti-inflammatory agent is selected from the group consisting of panthenol, α-bisabolol, betaine, monoammonium glycyrrhizate (MAG), lipochroman, tocopheryl acetate, phytosphingosine, extracts of green tea, extracts of *Sophora flavescens* root, extracts of chamomile (e.g. *Anthemis nobilis),* extracts of *Aloe vera,* extracts of *Echinacea,* extracts of willow bark, extracts of willow herb, extracts of almond, extracts of oats, glycyrrhizic acid, extracts of *Kola,* extracts of red clover, salicylic acid, xymeninic acid, turmeric, and combinations thereof,
wherein in (1), (2), (3) and (4), the polyphenolic antioxidant agent is selected from the group consisting of extracts of: green tea (e.g. green leaves of *Camellia sinensis*), extracts of mulberry (e.g. *Morus alba*), ginseng (e.g. *Panax ginseng),* raspberry, oregano (e.g. *Origanum vulgare),* white tea (e.g. *Camellia sinensis*), red tea, Mohani tea, black tea, Oolong tea, yellow tea, jasmine tea, Pu Erh tea, blueberry (e.g. *Vaccinium cyanococcus*), French maritime pine bark (e.g. *Pinus pinaster,* sold under the tradename of Pycnogenol), rosemary (e.g. *Rosmarinus officialis*), grape, including grape seed (e.g. *Vitis vinifera*), fennel (e.g. *Foeniculi fructus), Caragana sinica,* majaoram (e.g. *Origanum majorana*), crocus (e.g. *Crocus sativus),* apple (e.g. *Malus domestica*), coffee, green coffee, cherry (e.g. *Prunus avium*), snow algae (e.g. *Chlamydomonas nivalis),* Emblica (e.g. *Pyllanthus Emblica),* ginkgo (e.g. *Ginkgo biloba),* moringa (e.g. *Moringa oleilera*), ginger, magnolia (e.g. *Magnolioideae virginiana*), French saffron, edelweiss (e.g. *Leontopodium alpinium*), white lotus (e.g. nymphaea alba), turmeric root, marshmallow (e.g. *Althaea officianlis*), burdock (e.g. *Arctium lappa*), bilberry (e.g. *Vaccinium myrtillus),* cranberry (e.g. *Vaccinium oxycoccus*), pomegranate (e.g. *Punica granatum),* sage (e.g. *Salvia officianlis*), thyme (e.g. *Thymus vulgaris*), sunflower (e.g. *Helianthus annus*), wild carrot (e.g. *Daucus carota*), hop (e.g. *Humulus lupulus),* witch hazel (e.g. *Hamamelis),* oak (e.g. *Quercus),* Camellia (e.g. *Theacea*), red clover (e.g. *Tritolium pratense),* flax (e.g. *Linium usitatissiumum),* lemon (e.g. *Citrus limon),* birch (e.g. *Betula),* cornflower (e.g. *Centaurea cyanus),* geranium, polygonum, soy (e.g. *Glycine max),* Sophora (e.g. *Sophora flavescens)* and combinations thereof.

In one embodiment, the kit of the invention comprises:
1) a cleansing composition, the cleansing composition comprising: (i) a cosmetically acceptable carrier; (ii) one or more surfactants; (iii) one or more anti-inflammatory agent; (iv) one or more antioxidant agent selected from polyphenolic antioxidant agents and antioxidant vitamins; (v) one or more skin-soothing agent;
2) a skin balancing composition, the skin balancing composition comprising: (i) a cosmetically acceptable carrier; (ii) *Ophiopogon japonicas* extract; (iii) one or more antioxidant agent selected from polyphenolic antioxidant agents and antioxidant vitamins; (iv) one or more sebum control agent selected from *Rosa canina* extract and sphingosine compounds;
3) a daytime protective composition, the daytime protective composition comprising: (i) a cosmetically acceptable carrier; (ii) one or more UV-filter; (iii) one or more antioxidant agent selected from polyphenolic antioxidant agents and antioxidant vitamins; (iv) one or more skin-soothing agent;
4) an overnight treatment composition, the overnight treatment composition comprising: (i) a cosmetically acceptable carrier; (ii) one or more polyphenolic antioxidant agent; (iii) one or more antioxidant vitamin; (iv) one or more skin-soothing agent,
wherein in (1), the anti-inflammatory agent is selected from the group consisting of panthenol, α-bisabolol, betaine, monoammonium glycyrrhizate (MAG), lipochroman, tocopheryl acetate, phytosphingosine, extracts of green tea, extracts of *Sophora flavescens* root, extracts of chamomile (e.g. *Anthemis nobilis),* extracts of *Aloe vera,* extracts of *Echinacea,* extracts of willow bark, extracts of willow herb, extracts of almond, extracts of oats, glycyrrhizic acid, extracts of *Kola,* extracts of red clover, salicylic acid, xymeninic acid, turmeric, and combinations thereof,
wherein in (1), (2), (3) and (4), the polyphenolic antioxidant agent is selected from the group consisting of extracts of: green tea (e.g. green leaves of *Camellia sinensis*), extracts of mulberry (e.g. *Morus alba*), ginseng (e.g. *Panax ginseng*), raspberry, oregano (e.g. *Origanum vulgare),* white tea (e.g. *Camellia sinensis*), red tea, Mohani tea, black tea, Oolong tea, yellow tea, jasmine tea, Pu Erh tea, blueberry (e.g. *Vaccinium cyanococcus*), French maritime pine bark (e.g. *Pinus pinaster,* sold under the tradename of Pycnogenol), rosemary (e.g. *Rosmarinus officialis*), grape, including grape seed (e.g. *Vitis vinifera*), fennel (e.g. *Foeniculi fructus*), *Caragana sinica,* majaoram (e.g. *Origanum majorana*), crocus (e.g. *Crocus sativus),* apple (e.g. *Malus domestica*), coffee, green coffee, cherry (e.g. *Prunus avium*), snow algae (e.g. *Chlamydomonas nivalis*), Emblica (e.g. *Pyllanthus emblica),* ginkgo (e.g. *Ginkgo biloba),* moringa (e.g. *Moringa oleilera*), ginger, magnolia (e.g. *Magnolioideae virginiana*), French saffron, edelweiss (e.g. *Leontopodium alpinium*), white lotus (e.g. nymphaea alba), turmeric root, marshmallow (e.g. *Althaea officianlis*), burdock (e.g. *Arctium lappa*), bilberry (e.g. *Vaccinium myrtillus),* cranberry (e.g. *Vaccinium oxycoccus),* pomegranate (e.g. *Punica granatum),* sage (e.g. *Salvia officianlis*), thyme (e.g. *Thymus vulgaris*), sunflower (e.g. *Helianthus annus*), wild carrot (e.g. *Daucus carota*), hop (e.g. *Humulus lupulus),* witch hazel (e.g. *Hamamelis),* oak (e.g. *Quercus),* Camellia (e.g. *Theacea*), red clover (e.g. *Tritolium pratense*), flax (e.g. *Linium usitatissiumum),* lemon (e.g. *Citrus limon),* birch (e.g. *Betula),* cornflower (e.g. *Centaurea cyanus),* geranium, polygonum, soy (e.g. *Glycine max),* Sophora (e.g. *Sophora flavescens)* and combinations thereof,
wherein in (1), (2), (3) and (4), the antioxidant vitamin is be selected from the group consisting of vitamin C (ascorbic acid), vitamin E (tocotrienol and/or tocopherol) and beta carotene, derivatives thereof, and combinations thereof,
wherein in (1), (3) and (4) the skin-soothing agent is selected from the group consisting of glycerin, butylene glycol, allantoin, shea butter, sodium hyaluronate, bisaccharide gum-1, jojoba oil, isononyl isononanoate, carnauba wax, Aloe Vera, chamomile, comfrey, oatsmeal, coconut oil, honey, cocoa butter, almond oil and combinations thereof.

In one embodiment, the kit of the invention comprises:
1) a cleansing composition, the cleansing composition comprising: (i) a cosmetically acceptable carrier; (ii) one or more surfactants; (iii) one or more anti-inflammatory agent; (iv) one or more antioxidant agent selected from polyphenolic antioxidant agents and antioxidant vitamins; (v) one or more skin-soothing agent;
2) a skin balancing composition, the skin balancing composition comprising: (i) a cosmetically acceptable carrier; (ii) *Ophiopogon japonicas* extract; (iii) one or more antioxidant agent selected from polyphenolic antioxidant agents and antioxidant vitamins; (iv) one or more sebum control agent selected from *Rosa canina* extract and sphingosine compounds;
3) a daytime protective composition, the daytime protective composition comprising: (i) a cosmetically acceptable carrier; (ii) one or more UV-filter; (iii) one or more antioxidant agent selected from polyphenolic antioxidant agents and antioxidant vitamins; (iv) one or more skin-soothing agent;
4) an overnight treatment composition, the overnight treatment composition comprising: (i) a cosmetically acceptable carrier; (ii) one or more polyphenolic antioxidant agent; (iii) one or more antioxidant vitamin; (iv) one or more skin-soothing agent,
wherein in (1), the anti-inflammatory agent is selected from the group consisting of panthenol, α-bisabolol, betaine, monoammonium glycyrrhizate (MAG), lipochroman, tocopheryl acetate, phytosphingosine, extracts of green tea, extracts of *Sophora flavescens* root, extracts of chamomile (e.g. *Anthemis nobilis),* extracts of *Aloe vera,* extracts of *Echinacea,* extracts of willow bark, extracts of willow herb, extracts of almond, extracts of oats, glycyrrhizic acid, extracts of *Kola,* extracts of red clover, salicylic acid, xymeninic acid, turmeric, and combinations thereof,
wherein in (1), (2), (3) and (4), the polyphenolic antioxidant agent is selected from the group consisting of extracts of: green tea (e.g. green leaves of *Camellia sinensis*), extracts of mulberry (e.g. *Morus alba*), ginseng (e.g. *Panax ginseng),* raspberry, oregano (e.g. *Origanum vulgare),* white tea (e.g. *Camellia sinensis*), red tea, Mohani tea, black tea, Oolong tea, yellow tea, jasmine tea, Pu Erh tea, blueberry (e.g. *Vaccinium cyanococcus*), French maritime pine bark (e.g. *Pinus pinaster,* sold under the tradename of Pycnogenol), rosemary (e.g. *Rosmarinus officialis*), grape, including grape seed (e.g. *Vitis vinifera*), fennel (e.g. *Foeniculi fructus), Caragana sinica,* majaoram (e.g. *Origanum majorana*), crocus (e.g. *Crocus sativus),* apple (e.g. *Malus domestica*), coffee, green coffee, cherry (e.g. *Prunus avium*), snow algae (e.g. *Chlamydomonas nivalis),* Emblica (e.g. *Pyllanthus emblica),* ginkgo (e.g. *Ginkgo biloba),* moringa (e.g. *Moringa oleilera*), ginger, magnolia (e.g. *Magnolioideae virginiana*), French saffron, edelweiss (e.g. *Leontopodium alpinium*), white lotus (e.g. nymphaea alba), turmeric root, marshmallow (e.g. *Althaea officianlis*), burdock (e.g. *Arctium lappa*), bilberry (e.g. *Vaccinium myrtillus),* cranberry (e.g. *Vaccinium oxycoccus),* pomegranate (e.g. *Punica granatum),* sage (e.g. *Salvia officianlis*), thyme (e.g. *Thymus vulgaris*), sunflower (e.g. *Helianthus annus*), wild carrot (e.g. *Daucus carota*), hop (e.g. *Humulus lupulus),* witch hazel (e.g. *Hamamelis),* oak (e.g. *Quercus),* Camellia (e.g. *Theacea*), red clover (e.g. *Tritolium pratense*), flax (e.g. *Linium usitatissiumum),* lemon (e.g. *Citrus limon),* birch (e.g. *Betula),* cornflower (e.g. *Centaurea cyanus),* geranium, polygonum, soy (e.g. *Glycine max),* Sophora (e.g. *Sophora flavescens)* and combinations thereof,
wherein in (1), (2), (3) and (4), the antioxidant vitamin is be selected from the group consisting of vitamin C (ascorbic acid), vitamin E (tocotrienol and/or tocopherol) and beta carotene, derivatives thereof, and combinations thereof,
wherein in (1), (3) and (4) the skin-soothing agent is selected from the group consisting of glycerin, butylene glycol, allantoin, shea butter, sodium hyaluronate, bisaccharide gum-1, jojoba oil, isononyl isononanoate, carnauba wax, Aloe Vera, chamomile, comfrey, oatsmeal, coconut oil, honey, cocoa butter, almond oil and combinations thereof,
wherein in (2), the sphingosine compound is selected from the group consisting of dihydrosphingosine (sphinganine), phytosphingosine, salicyloyl phytosphingosine (phytosphingosine SLC), phytosphingosine hydrochloride (phytosphingosine HCl), sphingonine hydroxysphingosine, and sphingosine-1-phosphate, and combinations thereof.

In one embodiment, the kit of the invention comprises:
1) a cleansing composition, the cleansing composition comprising: (i) a cosmetically acceptable carrier; (ii) one or more surfactants; (iii) one or more anti-inflammatory agent; (iv) one or more antioxidant agent selected from polyphenolic antioxidant agents and antioxidant vitamins; (v) one or more skin-soothing agent;
2) a skin balancing composition, the skin balancing composition comprising: (i) a cosmetically acceptable carrier; (ii) *Ophiopogon japonicas* extract; (iii) one or more antioxidant agent selected from polyphenolic antioxidant agents and antioxidant vitamins; (iv) one or more sebum control agent selected from *Rosa canina* extract and sphingosine compounds;
3) a daytime protective composition, the daytime protective composition comprising: (i) a cosmetically acceptable carrier; (ii) one or more UV-filter; (iii) one or more antioxidant agent selected from polyphenolic antioxidant agents and antioxidant vitamins; (iv) one or more skin-soothing agent;
4) an overnight treatment composition, the overnight treatment composition comprising: (i) a cosmetically acceptable carrier; (ii) one or more polyphenolic antioxidant agent; (iii) one or more antioxidant vitamin; (iv) one or more skin-soothing agent,
wherein in (1), the anti-inflammatory agent is selected from the group consisting of panthenol, α-bisabolol, betaine, monoammonium glycyrrhizate (MAG), lipochroman, tocopheryl acetate, phytosphingosine, extracts of green tea, and combinations thereof, and optionally is present in an amount of from about 0.0001% to about 10% by weight of the cleansing composition (e.g. about 0.0001% to about 5%, about 0.0005% to about 3%),
wherein in (1), (2), (3) and (4), the polyphenolic antioxidant agent is selected from the group consisting of extracts of: green tea (e.g. green leaves of *Camellia sinensis*), Emblica (e.g. *Pyllanthus emblica*), ginkgo (e.g. *Ginkgo biloba),* mulberry (e.g. *Morus alba*), ginseng (e.g. *Panax ginseng),* and Sophora (e.g. *Sophora flavescens)* and combinations thereof, and optionally is present in an amount of from about 0.0001% to about 20% by weight of the respective composition (e.g. about 0.0001% to about 10%, about 0.0001% to about 5%),
wherein in (1), (2), (3) and (4), the antioxidant vitamin is be selected from the group consisting of vitamin C (ascorbic acid), vitamin E (tocotrienol and/or tocopherol) and combinations thereof, and optionally is present in an amount of from about 0.01% to about 20% by weight of the respective composition (e.g. about 0.01% to about 10%, about 0.05% to about 5%),
wherein in (1), (3) and (4) the skin-soothing agent is selected from the group consisting of glycerin, butylene glycol, shea butter, sodium hyaluronate, bisaccharide gum-1, jojoba oil, isononyl isononanoate, carnauba wax, and combinations thereof, and optionally is present in an amount of from about 0.01% to about 50% by weight of the respective composition (e.g. about 0.1% to about 40%, about 1% to about 35%),
wherein in (2), the sphingosine compound is selected from the group consisting of dihydrosphingosine (sphinganine), phytosphingosine, phytosphingosine hydrochloride (phytosphingosine HCl), and combinations thereof, and optionally is present in an amount of from about 0.001% to about 10% by weight of the skin balancing composition (e.g. about 0.01% to about 5%, about 0.01% to about 0.5%).

In one embodiment, the kit of the invention comprises
1) a cleansing composition, the cleansing composition comprising: (i) a cosmetically acceptable carrier; (ii) one or more surfactants; (iii) one or more anti-inflammatory agent; (iv) one or more antioxidant agent selected from polyphenolic antioxidant agents and antioxidant vitamins; (v) one or more skin-soothing agent;
2) a skin balancing composition, the skin balancing composition comprising: (i) a cosmetically acceptable carrier; (ii) *Ophiopogon japonicas* extract; (iii) one or more antioxidant agent selected from polyphenolic antioxidant agents and antioxidant vitamins; (iv) one or more sebum control agent selected from *Rosa canina* extract and sphingosine compounds;
3) a daytime protective composition, the daytime protective composition comprising: (i) a cosmetically acceptable carrier; (ii) one or more UV-filter; (iii) one or more antioxidant agent selected from polyphenolic antioxidant agents and antioxidant vitamins; (iv) one or more skin-soothing agent;
4) an overnight treatment composition, the overnight treatment composition comprising: (i) a cosmetically acceptable carrier; (ii) one or more polyphenolic antioxidant agent; (iii) one or more antioxidant vitamin; (iv) one or more skin-soothing agent; and
5) a skin improvement composition in the form of an intensive moisture product, an anti-shine product or an acne treatment product,
wherein when present the intensive moisture product comprises: (i) a cosmetically acceptable carrier; (ii) *Ophiopogon japonicas* extract; (iii) one or more antioxidant agent selected from polyphenolic antioxidant agents and antioxidant vitamins; (iv) one or more skin-soothing agent,
wherein when present the anti-shine product comprises: (i) a cosmetically acceptable carrier; (ii) one or more silicone agent; (iii) one or more antioxidant agent selected from polyphenolic antioxidant agents and antioxidant vitamins; (iv) one or more skin-soothing agent, and
wherein when present the acne treatment product comprises: (i) a cosmetically acceptable carrier; (ii) one or more salicylic acid compound; (iii) one or more antioxidant agent selected from polyphenolic antioxidant agents and antioxidant vitamins; (iv) one or more anti-inflammatory agent.

In one embodiment, the kit of the invention comprises:
1) a cleansing composition, the cleansing composition comprising: (i) a cosmetically acceptable carrier; (ii) one or more surfactants; (iii) one or more anti-inflammatory agent; (iv) one or more antioxidant agent selected from polyphenolic antioxidant agents and antioxidant vitamins; (v) one or more skin-soothing agent;
2) a skin balancing composition, the skin balancing composition comprising: (i) a cosmetically acceptable carrier; (ii) *Ophiopogon japonicas* extract; (iii) one or more antioxidant agent selected from polyphenolic antioxidant agents and antioxidant vitamins; (iv) one or more sebum control agent selected from *Rosa canina* extract and sphingosine compounds;
3) a daytime protective composition, the daytime protective composition comprising: (i) a cosmetically acceptable carrier; (ii) one or more UV-filter; (iii) one or more antioxidant agent selected from polyphenolic antioxidant agents and antioxidant vitamins; (iv) one or more skin-soothing agent;
4) an overnight treatment composition, the overnight treatment composition comprising: (i) a cosmetically acceptable carrier; (ii) one or more polyphenolic antioxidant agent; (iii) one or more antioxidant vitamin; (iv) one or more skin-soothing agent;
5) a skin improvement composition in the form of an intensive moisture product, an anti-shine product or an acne treatment product,
wherein when present the intensive moisture product in (5) comprises: (i) a cosmetically acceptable carrier; (ii) *Ophiopogon japonicas* extract; (iii) one or more antioxidant agent selected from polyphenolic antioxidant agents and antioxidant vitamins; (iv) one or more skin-soothing agent,
wherein when present the anti-shine product in (5) comprises: (i) a cosmetically acceptable carrier; (ii) one or more silicone agent; (iii) one or more antioxidant agent selected from polyphenolic antioxidant agents and antioxidant vitamins; (iv) one or more skin-soothing agent, and
wherein when present the acne treatment product in (5) comprises: (i) a cosmetically acceptable carrier; (ii) one or more salicylic acid compound; (iii) one or more antioxidant agent selected from polyphenolic antioxidant agents and antioxidant vitamins; (iv) one or more anti-inflammatory agent
wherein in (1) and (5), the anti-inflammatory agent is selected from the group consisting of panthenol, α-bisabolol, betaine, monoammonium glycyrrhizate (MAG), lipochroman, tocopheryl acetate, phytosphingosine, extracts of green tea, and combinations thereof, and optionally is present in an amount of from about 0.0001% to about 10% by weight of the cleansing composition (e.g. about 0.0001% to about 5%, about 0.0005% to about 3%),
wherein in (1), (2), (3), (4), and (5) the polyphenolic antioxidant agent is selected from the group consisting of extracts of: green tea (e.g. green leaves of *Camellia sinensis*), Emblica (e.g. *Pyllanthus Emblica*), ginkgo (e.g. *Ginkgo biloba),* mulberry (e.g. *Morus alba*), ginseng (e.g. *Panax ginseng),* and Sophora (e.g. *Sophora flavescens)* and combinations thereof, and optionally is present in an amount of from about 0.0001% to about 20% by weight of the respective composition (e.g. about 0.0001% to about 10%, about 0.0001% to about 5%),
wherein in (1), (2), (3), (4) and (5), the antioxidant vitamin is be selected from the group consisting of vitamin C (ascorbic acid), vitamin E (tocotrienol and/or tocopherol) and combinations thereof, and optionally is present in an amount of from about 0.01% to about 20% by weight of the respective composition (e.g. about 0.01% to about 10%, about 0.05% to about 5%),
wherein in (1), (3), (4) and (5) the skin-soothing agent is selected from the group consisting of glycerin, butylene glycol, shea butter, sodium hyaluronate, bisaccharide gum-1, jojoba oil, isononyl isononanoate, carnauba wax, and combinations thereof, and optionally is present in an amount of from about 0.01% to about 50% by weight of the respective composition (e.g. about 0.1% to about 40%, about 1% to about 35%),
wherein in (2), the sphingosine compound is selected from the group consisting of dihydrosphingosine (sphinganine), phytosphingosine, phytosphingosine hydrochloride (phytosphingosine HCl), and combinations thereof, and optionally is present in an amount of from about 0.001% to about 10% by weight of the skin balancing composition (e.g. about 0.01% to about 5%, about 0.01% to about 0.5%).

The skilled person will understand that optional features of one embodiment or aspect of the invention may be applicable, where appropriate, to other embodiments or aspects of the invention.

There now follows by way of example only a description of the present invention with reference to the accompanying drawings, in which:
**Figure 1** shows a list of example cleansing compositions according to the invention;

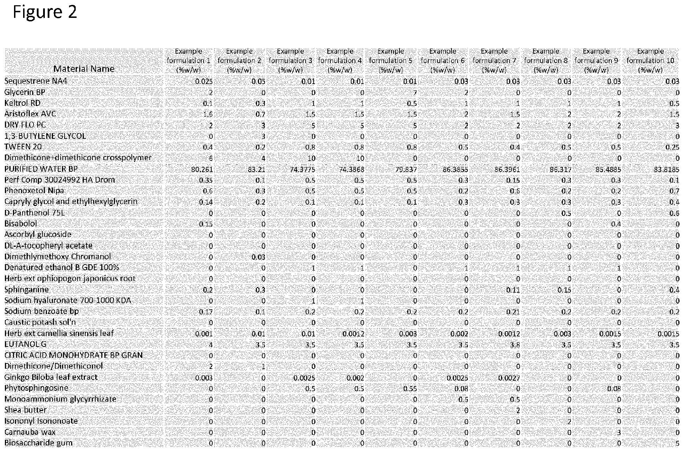

**Figure 2** shows a list of example skin-balancing compositions according to the invention;

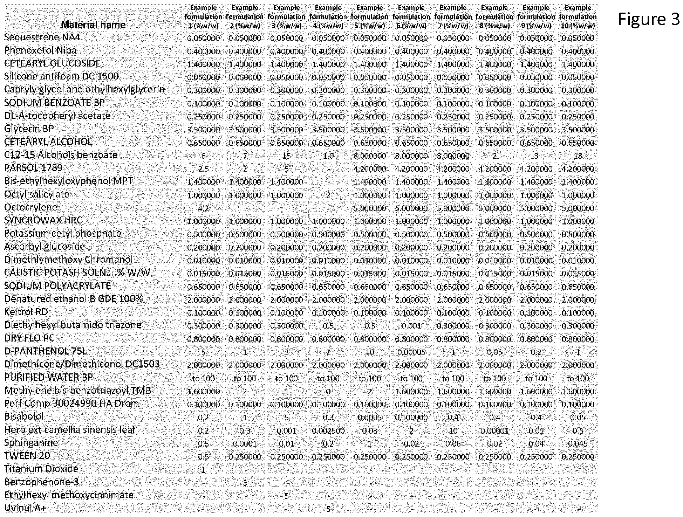

**Figure 3** shows a list of example daytime protective compositions according to the invention;

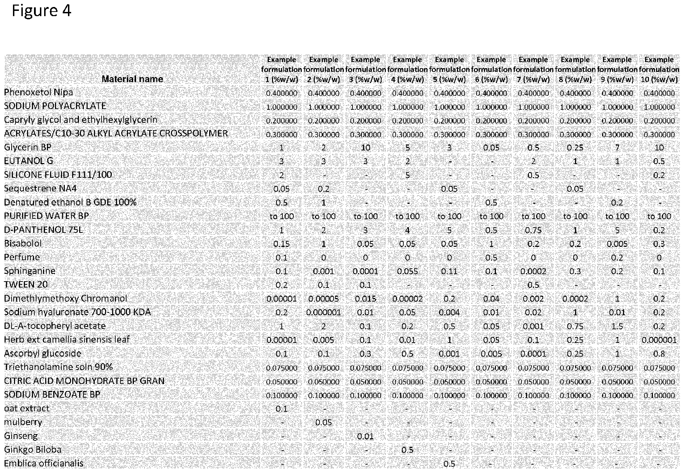

**Figure 4** shows a list of example overnight treatment compositions according to the invention;

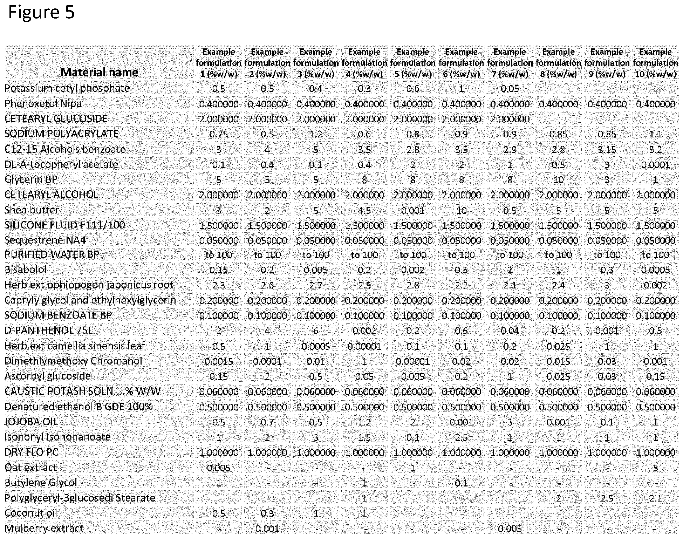

**Figure 5** shows a list of example intensive moisture products according to the invention;

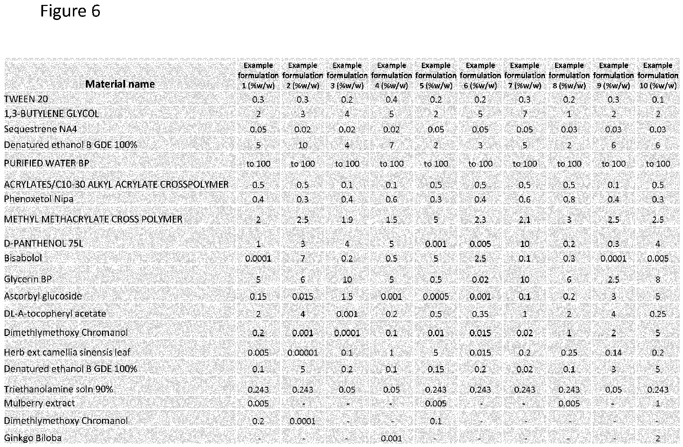

**Figure 6** shows a list of example anti-shine products according to the invention;

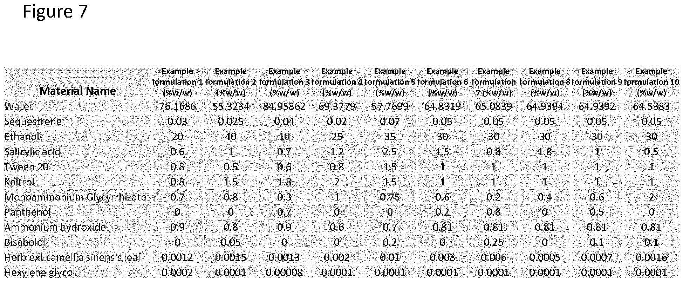

**Figure 7** shows a list of example acne treatment products according to the invention; and

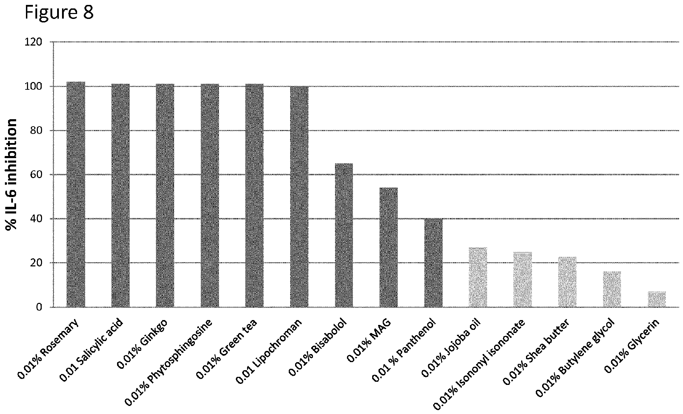

**Figure 8** is a graph showing the percentage IL-6 inhibition in skin cells in the presence of anti-inflammatory agents and in the presence of skin-soothing agents.

### Interleukin-6 (IL-6) assay

Interleukin-6 (IL-6) bioactivity in skin cells was measured. IL-6 is a cytokine and used as a biomarker for inflammation. Skin cells (keratinocytes or fibroblasts) were placed under UV stress to induce an IL-6 response in the presence of anti-inflammatory agents or skin-soothing agents to determine their activity in inhibiting an IL-6 response.

Cells (keratinocytes or fibroblasts) were taken from culture, seeded in a 96-well plate at a density of 5000 cells per well in cell growth media with supplements and left to incubate for 24 hours at 37°C. Determination of cell number for the purpose of plating was performed in accordance with standard methods known in the art.

After incubation, the cell growth media was replaced with 100µl of PBS without calcium and magnesium and containing the relevant concentration of agent(s) required. Controls include untreated irradiated and non-irradiated samples which require 100µl pure PBS without calcium and magnesium or containing a maximum of 0.1%. Dimethyl sulfoxide (DMSO) was used where necessary to dissolve the agent(s). Salicylic acid is the positive control for this assay.

The cells were incubated with the agents for 30 minutes at 37°C before being irradiated with a UV dose of 61,500 Joules/m2.

After irradiation, the PBS and agents were replaced with 100µl of pre-warmed (37°C) media without supplements and incubated for 24 hours at 37°C.

Media supernatant containing the Il-6 expressed from the cells was then collected and transferred to a fresh 96-well plate and stored at -20°C until the ELISA was performed.

The Il-6 ELISA was performed according to the manufacturer's protocol provided with the kit and as standard in the art.

A cell viability assay was performed on remaining cells where necessary to determine the cytotoxic effects of tested agents.

### Results

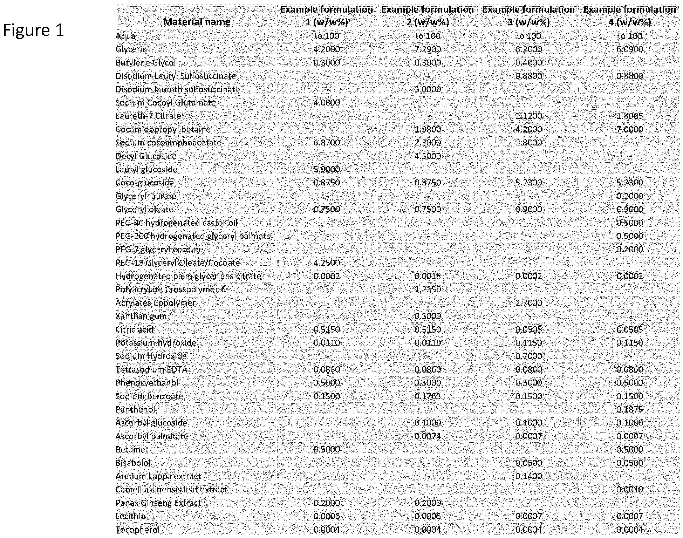

Figure 1 shows that the percentage IL-6 inhibition in skin cells in the presence of the anti-inflammatory agents was higher (at least 40% IL-6 inhibition) than in the presence of the skin-soothing agents (which achieved no more than 30% IL-6 inhibition).

## Claims

1. A kit of cosmetic compositions, the kit comprising:
(1) a cleansing composition, the cleansing composition comprising: (i) a cosmetically acceptable carrier; (ii) one or more surfactants; (iii) one or more anti-inflammatory agent; and (iv) one or more antioxidant agent selected from polyphenolic antioxidant agents and antioxidant vitamins;
(2) a skin balancing composition, the skin balancing composition comprising: (i) a cosmetically acceptable carrier; (ii) a skin-barrier enhancing agent selected from the group consisting of extracts of *Ophiopogon (e.g. Ophiopogon japonicas),* extracts of Aloe (e.g. *Aloe Vera*), extracts of marshmallow (e.g. *Althaea officianlis*), extracts of Allium (e.g. *Allium cepa*), extracts of tuberose (e.g. *Polianthes tuberosa*), extracts of dandelion, extracts of seaweed (e.g. *Laminaria digitate),* ceramides, and combinations thereof; (iii) one or more antioxidant agent selected from polyphenolic antioxidant agents and antioxidant vitamins; (iv) one or more sebum control agent selected from the group consisting of sphingosine compounds, *Rosa canina* extract, tea tree extract (e.g. *Melaleuca alternifolia*), witch hazel extract (e.g. *Hamamelis virginiana*), and combinations thereof; and
(3) a daytime protective composition, the daytime protective composition comprising: (i) a cosmetically acceptable carrier; (ii) one or more UV-filter; (iii) one or more antioxidant agent selected from polyphenolic antioxidant agents and antioxidant vitamins; (iv) one or more skin-soothing agent.

2. The kit of claim 1, wherein the kit further comprises (4) an overnight treatment composition, the overnight treatment composition comprising: (i) a cosmetically acceptable carrier; (ii) one or more polyphenolic antioxidant agent; (iii) one or more antioxidant vitamin; and (iv) one or more skin-soothing agent.

3. The kit of any one of the preceding claims, wherein the cleansing composition further comprises (v) a skin-soothing agent.

4. The kit of any one of the preceding claims, wherein the one or more sebum control agent (iv) is selected from sphingosine compound, *Rosa canina* extract and combinations thereof.

5. The kit of any one of the preceding claims, wherein the kit further comprises (5) a skin improvement composition in the form of an intensive moisture product, an anti-shine product or an acne treatment product.

6. The kit of claim 5, wherein
the intensive moisture product comprises: (i) a cosmetically acceptable carrier; (ii) a skin-barrier enhancing agent selected from the group consisting of extracts of *Ophiopogon (e.g. Ophiopogon japonicas),* extracts of Aloe (e.g. *Aloe Vera*), extracts of marshmallow (e.g. *Althaea officianlis*), extracts of Allium (e.g. *Allium cepa*), extracts of tuberose (e.g. *Polianthes tuberosa*), extracts of dandelion, extracts of seaweed (e.g. *Laminaria digitate),* ceramides, and combinations thereof; (iii) one or more antioxidant agent selected from polyphenolic antioxidant agents and antioxidant vitamins; (iv) one or more skin-soothing agent,
the anti-shine product comprises: (i) a cosmetically acceptable carrier; (ii) one or more silicone agent; (iii) one or more antioxidant agent selected from polyphenolic antioxidant agents and antioxidant vitamins; (iv) one or more skin-soothing agent,
the acne treatment product comprises: (i) a cosmetically acceptable carrier; (ii) one or more salicylic acid compound; (iii) one or more antioxidant agent selected from polyphenolic antioxidant agents and antioxidant vitamins; (iv) one or more anti-inflammatory agent.

7. The kit of any one of the preceding claims, wherein the skin-barrier enhancing agent (ii) in the skin balancing composition is *Ophiopogon japonicas* extract and/or the kit of claim 6 wherein the skin-barrier enhancing agent (ii) in the intensive moisture product is *Ophiopogon japonicas* extract.

8. The kit of any preceding claim, wherein in compositions (1) and (5), the anti-inflammatory agent:
(i) is selected from the group consisting of panthenol, α-bisabolol, betaine, monoammonium glycyrrhizate (MAG), lipochroman, tocopheryl acetate, phytosphingosine, extracts of green tea, extracts of *Sophora flavescens* root, extracts of chamomile (e.g. *Anthemis nobilis),* extracts of *Aloe vera,* extracts of *Echinacea,* extracts of willow bark, extracts of willow herb, extracts of almond, extracts of oats, glycyrrhizic acid, extracts of *Kola,* extracts of red clover, salicylic acid, xymeninic acid, turmeric, and combinations thereof; and/or
(ii) is present in an amount of:
(a) from about 0.0001% to about 10% by weight of the respective composition; or
(b) from about 0.0001% to about 5% by weight of the respective composition; or
(c) from about 0.005% to about 3% by weight of the respective composition.

9. The kit of any one of the preceding claims, wherein in compositions (1), (2), (3), (4) and (5), the polyphenolic antioxidant agent:
(i) is selected from the group consisting of extracts of: green tea, extracts of mulberry, ginseng, raspberry, oregano, white tea, red tea, Mohani tea, black tea, Oolong tea, yellow tea, jasmine tea, Pu Erh tea, blueberry, French maritime pine bark, rosemary, grape, including grape seed, fennel, *Caragana sinica,* majaoram, crocus, apple, coffee, green coffee, cherry, snow algae, Emblica, ginkgo, moringa, ginger, magnolia, French saffron, edelweiss, white lotus, turmeric root, marshmallow, burdock, bilberry, cranberry, pomegranate, sage, thyme, sunflower, wild carrot, hop, witch hazel, oak, Camellia, red clover, flax, lemon, birch, cornflower, geranium, polygonum, soy, Sophora and combinations thereof; and/or
(ii) is present in an amount of:
(a) from about 0.0001% to about 20% by weight of the respective composition; or
(b) from about 0.0001% to about 10% by weight of the respective composition; or
(c) from about 0.0001% to about 5% by weight of the respective composition.

10. The kit of any one of the preceding claims, wherein in compositions (1), (2), (3) (4) and (5), the antioxidant vitamin:
(i) is selected from the group consisting of vitamin C (ascorbic acid), vitamin E (tocotrienol and/or tocopherol) and beta carotene, derivatives thereof, and combinations thereof; and/or
(ii) is present in an amount of:
(a) from about 0.01% to about 20% by weight of the respective composition; or
(b) from about 0.01% to about 10% by weight of the respective composition; or
(c) from about 0.05% to about 5% by weight of the respective composition.

11. The kit of any one of the preceding claims, wherein in compositions (1), (3) (4) and (5) the skin-soothing agent:
(i) is selected from the group consisting of glycerin, butylene glycol, allantoin, shea butter, sodium hyaluronate, bisaccharide gum-1, jojoba oil, isononyl isononanoate, carnauba wax, Aloe Vera, chamomile, comfrey, oatsmeal, coconut oil, honey, cocoa butter, almond oil and combinations thereof; and/or
(ii) is present in an amount of:
(a) from about 0.01% to about 50% by weight of the respective composition; or
(b) from about 0.1% to about 40% by weight of the respective composition; or
(c) from about 1% to about 35% by weight of the respective composition.

12. The kit of any one of the preceding claims, wherein in composition (2), the sphingosine compound:
(i) is selected from the group consisting of dihydrosphingosine (sphinganine), phytosphingosine, salicyloyl phytosphingosine (phytosphingosine SLC), phytosphingosine hydrochloride (phytosphingosine HCl), sphingonine hydroxysphingosine, and sphingosine-1-phosphate, and combinations thereof; and/or
(ii) is present in an amount of:
(a) from about 0.001% to about 10% by weight of the skin-balancing composition; or
(b) from about about 0.01% to about 5% by weight of the skin-balancing composition; or
(c) from about 0.01% to about 0.5% by weight of the skin-balancing composition.

13. A method of using the kit as defined in any one of the preceding claims, in which each of the cosmetic compositions is applied as a topical application on the skin at least once a day.

14. Use of the cleansing composition as defined in any one of the preceding claims to cleanse the skin twice daily.

15. A method of cosmetically treating skin damage as a result of pollution insult, or of cosmetically preventing the detrimental effects of pollution to the skin, said method comprising using the kit as defined in any one of claims 1 to 13, in which each of the cosmetic compositions is applied as a topical application on the skin at least once a day.
